# EUROPEAN PATENT SPECIFICATION

(11) **EP 4 157 182 B1**
(45) Date of publication and mention of the grant of the patent: **04.11.2026**
(21) Application number: 21732710.5
(22) Date of filing: 20.05.2021
(51) Int. Cl.: A61F 13/514

(54) **ABSORBENT ARTICLES HAVING LAMINATES EXHIBITING HIGHLY RECOGNIZABLE PATTERNS AND VIBRANT GRAPHICS**
ABSORBIERENDE ARTIKEL MIT LAMINATEN MIT HOCH ERKENNBAREN MUSTERN UND VIBRANTEN GRAFIKEN
ARTICLES ABSORBANTS AYANT DES STRATIFIÉS PRÉSENTANT DES MOTIFS HAUTEMENT RECONNAISSABLES ET DES ÉLÉMENTS GRAPHIQUES VIBRATOIRES

(30) Priority: 28.05.2020 US 202063031059 P
(43) Date of publication of application: 05.04.2023
(73) Proprietor: The Procter & Gamble Company, Cincinnati, OH 45202 (US)
(72) Inventor: GIOVANNI, Sara, L., Cincinnati, Ohio 45202 (US); ASHRAF, Arman, Cincinnati, Ohio 45202 (US); JAYAKARAN, Jacob, Cincinnati, Ohio 45202 (US); SANTA HORNEDO, Kristian, Rafael, Cincinnati, Ohio 45202 (US); SCHIANO, Danielle, Cincinnati, Ohio 45202 (US); ALVARADO, Keith, Cincinnati, Ohio 45202 (US); WADE, Sarah, M., Cincinnati, Ohio 45202 (US); ARORA, Kelyn, A., Cincinnati, Ohio 45202 (US); HAMMONS, John, L., Cincinnati, Ohio 45202 (US)
(74) Representative: P&G Patent Germany
(86) International application number: PCT/US2021/033285
(87) International publication number: WO 2021/242593

(56) References cited:
- US-A1- 2009 012 491
- US-A1- 2017 105 881
- US-A1- 2020 100 956
- US-B1- 6 569 136

## Description

### FIELD

The present disclosure is directed to absorbent articles comprising laminates of an outer cover material comprising a pattern or patterns in combination with a backsheet comprising one or more color graphics for improved pattern recognition and overall vibrant graphics perception.

### BACKGROUND

Absorbent articles are used in the hygiene industry to contain and absorb bodily exudates (i.e., urine, bowel movements, and menses) in infants, toddlers, children, and adults. Absorbent articles may include, but not be limited to, diapers, pants, adult incontinence products, feminine care products, and absorbent pads. In some instances, one or more patterns are formed into a topsheet, landing zone, and/or an outer cover material of the absorbent article. When underlying components, such as backsheets, are free of graphics or comprise graphics composed of muted colors, the patterns in the topsheet, landing zone, and/or outer cover material are quite difficult to discern. This may be especially true when the patterns comprise relatively large, discrete, recognizable indicia. Additionally, when outer cover nonwoven materials, landing zones, or topsheets are planar, non-patterned webs, the underlying graphics are mottled, uniformly muted, and/or uniformly masked to a degree from the view of a consumer observing through the topsheets, landing zones, or the outer cover nonwoven materials.

US 6 569 136 B1 relates to disposable absorbent articles comprising a backsheet comprised of a microporous polymer film printed with multicolored graphics and a nonwoven material laminated to the film. The film has a "b" value between about 0.0 and about 0.5 and exhibits less than about 4.0% thermal shrinkage at about 50° C and about 50% relative humidity for one week.

US 2009/012491 A1 is concerned with pantiliners having a fluid impermeable barrier disposed between the absorbent core and the nonwoven backsheet. The fluid impermeable barrier includes a colored pattern visible through at least one of the nonwoven topsheet or the backsheet.

US 2017/105881 A1 discloses an absorbent article comprising an outer blouse layer formed of a nonwoven web. The blouse layer has a blouse layer surface area and the blouse layer is attached to the article at less than 50 percent of the blouse layer surface area. The material of the blouse layer and underlying layer may be colored to create a visual contrast.

US 2020/100956 A1 relates to nonwoven webs for absorbent articles. The web comprisess a first surface, a second surface, and a visually discernible pattern of three-dimensional features on the first surface or the second surface. The three-dimensional features comprise one or more first regions and a plurality of second regions. The one or more first regions are different than the plurality of second regions in a value of an average intensive property.

US 2019/060135 A1 discusses methods and apparatus for assembling an absorbent article. One or more sensors are used to detect registration features based on the features of each component of the absorbent article. The sensor communicates with a controller to change the speed and position of the components to assemble the absorbent article such that features are positioned with respect to one another.

As such, outer cover materials, landing zones, topsheets, and backsheet graphics should be improved.

### SUMMARY

The invention provides an absorbent article as defined in the claims.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin;
Fig. 13A is a plan view of a backsheet comprising graphics with a garment-facing surface facing the viewer;
Fig. 13B is a plan view of a backsheet comprising graphics with a garment-facing surface facing the viewer;
Fig. 14 is a plan view of a backsheet comprising graphics with a wearer-facing surface facing the viewer;
Fig. 15 is a plan view of an outer cover material comprising an embossed pattern;
Fig. 16 is a plan view of a topsheet comprising an embossed pattern;
Fig. 17A is a plan view of a backsheet comprising one or more graphics with the garment-facing surface facing the viewer;
Fig. 17B is a plan view of an outer cover material comprising an embossed pattern;
Fig. 17C is a plan view of an outer cover material-backsheet laminate comprising the outer cover material of Fig. 17B overlaying the backsheet of Fig. 17A;
Fig. 18A is a plan view of a backsheet comprising one or more graphics with the wearer-facing surface facing the viewer;
Fig. 18B is a plan view of a topsheet comprising an embossed pattern;
Fig. 18C is a plan view of a topsheet-backsheet laminate comprising the topsheet of Fig. 18B overlaying the backsheet of Fig. 18A;
Fig 19 is a plan view of an absorbent article comprising a topsheet comprising a plurality of apertures, wearer-facing surface facing the viewer, in a flat laid-out state;
Fig. 20 is a plan view of an outer cover material comprising a plurality of apertures with the garment-facing surface facing the viewer;
Figs. 21-23 are examples of stitch-like patterns for backsheets; and
Fig. 24 is a plan view of a liquid impermeable backsheet comprising one or more visually vibrant graphics.

### DETAILED DESCRIPTION

Various non-limiting forms of the present disclosure will now be described to provide an overall understanding of the principles of the structure, function, manufacture, and use of the absorbent articles having laminates exhibiting highly recognizable patterns and vibrant graphics disclosed herein. One or more examples of these non-limiting forms are illustrated in the accompanying drawings. Those of ordinary skill in the art will understand that the absorbent articles having laminates exhibiting highly recognizable patterns and vibrant graphics described herein and illustrated in the accompanying drawings are non-limiting example forms. The features illustrated or described in connection with one non-limiting form may be combined with the features of other non-limiting forms. Such modifications and variations are intended to be included within the scope of the present disclosure.

### General Description of an Absorbent Article

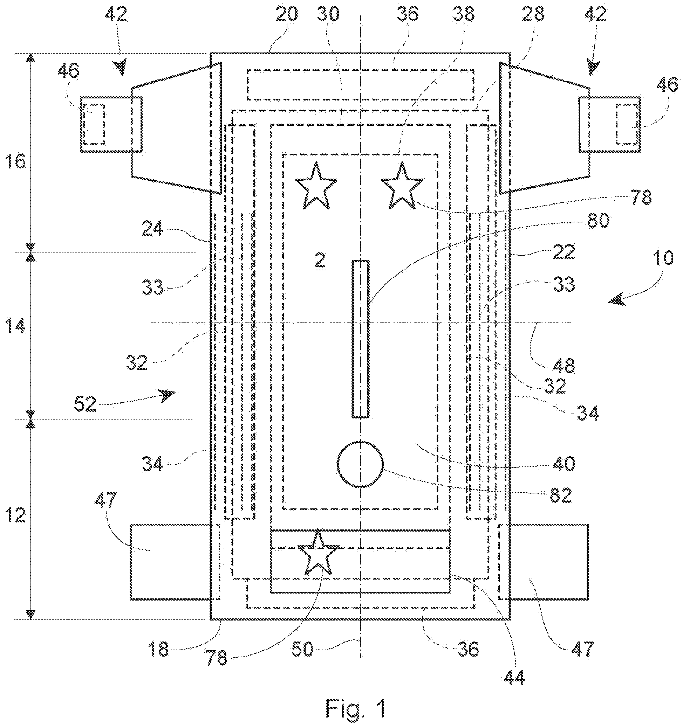

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction).

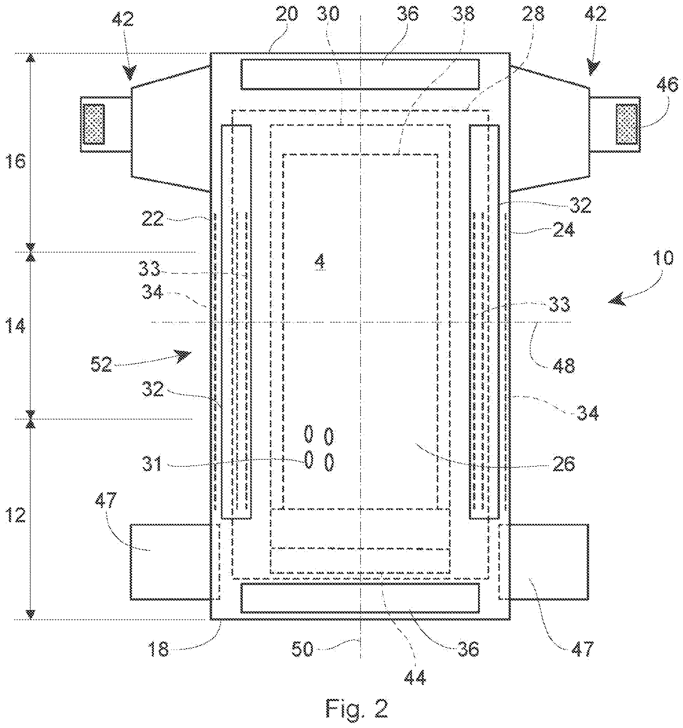

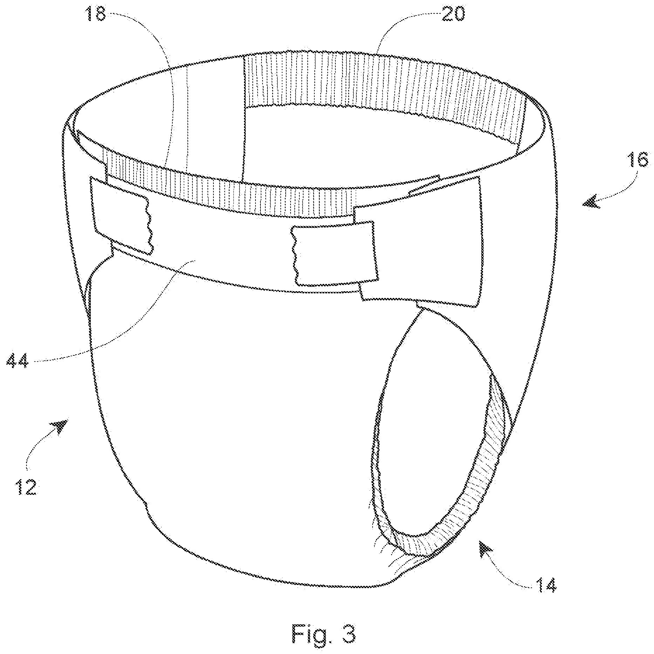

Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 comprises a liquid permeable topsheet 26, a liquid impermeable backsheet 28, and an absorbent core 30 positioned at least partially intermediate the topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing surface of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

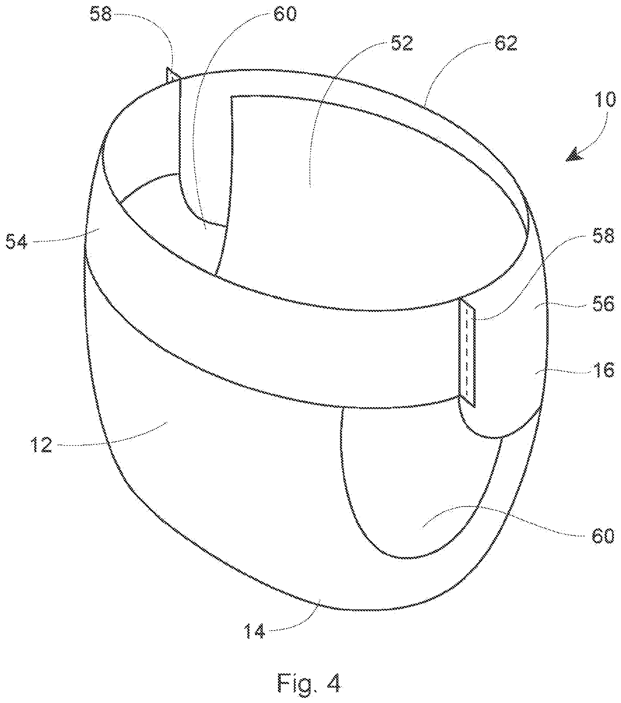

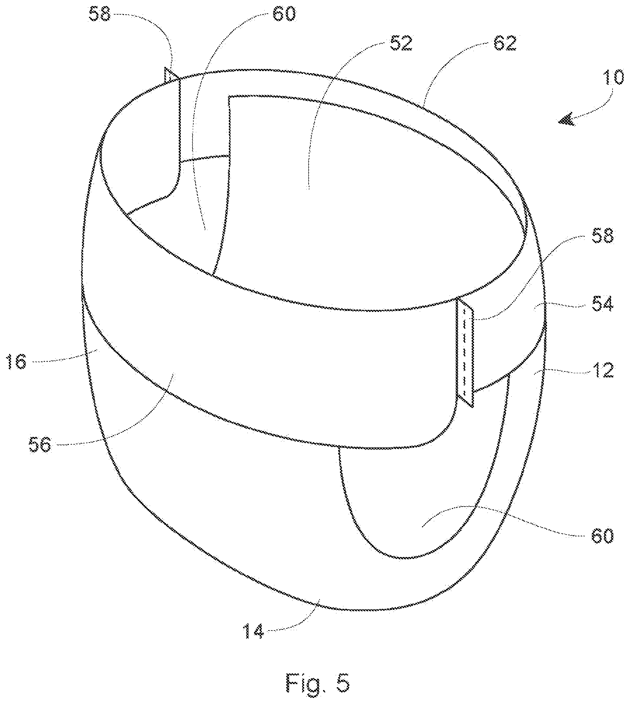

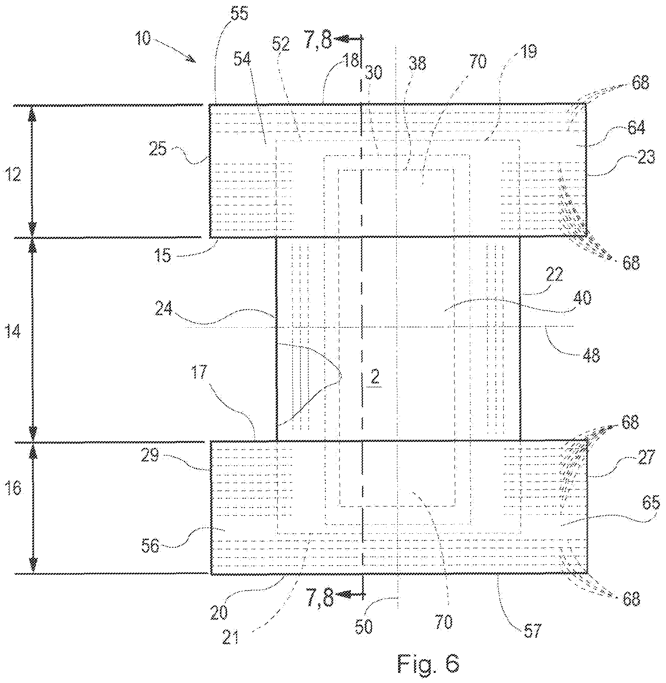

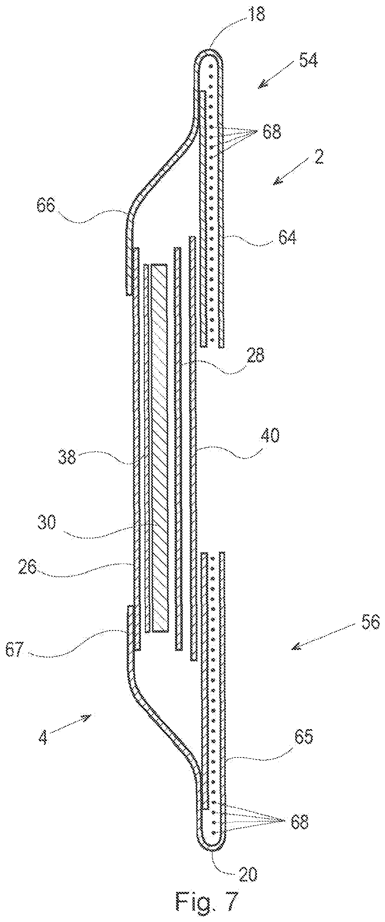

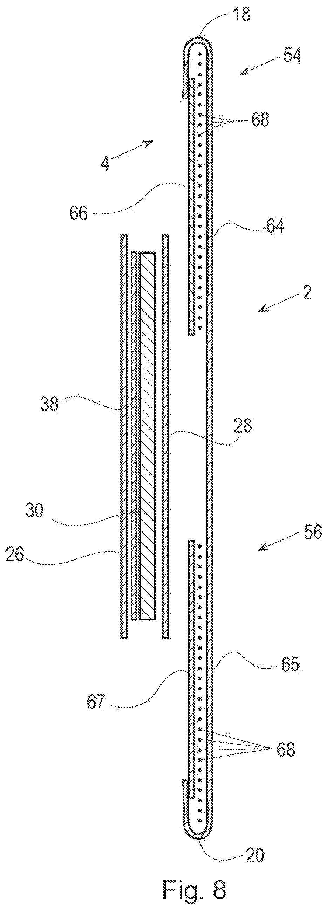

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. **In** other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31) and/or may have a plurality of embossments (e.g., a bond pattern), as discussed further below. The topsheet may have any suitable three-dimensional features. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The topsheet may comprise a pattern or patterns of apertures and/or embossments and may be positioned over one or more graphics on a backsheet to achieve one or more of the benefits of the present disclosure.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet. The backsheet comprises one or more graphics garment facing surface thereof, as described further herein.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features.

The outer cover material may comprise a pattern or patterns of apertures and/or embossments and may be positioned over one or more graphics on a backsheet to achieve one or more of the benefits of the present disclosure. One or more portions of the outer cover nonwoven materials may comprise one or more integral landing zones.

### Absorbent Core

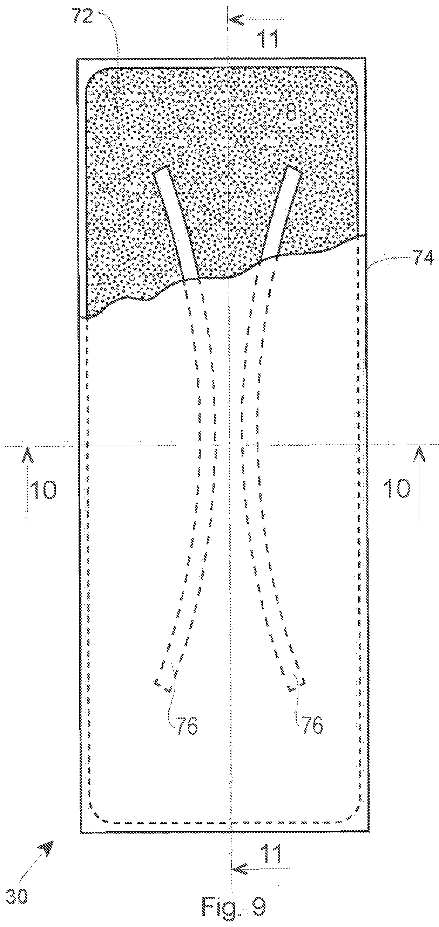

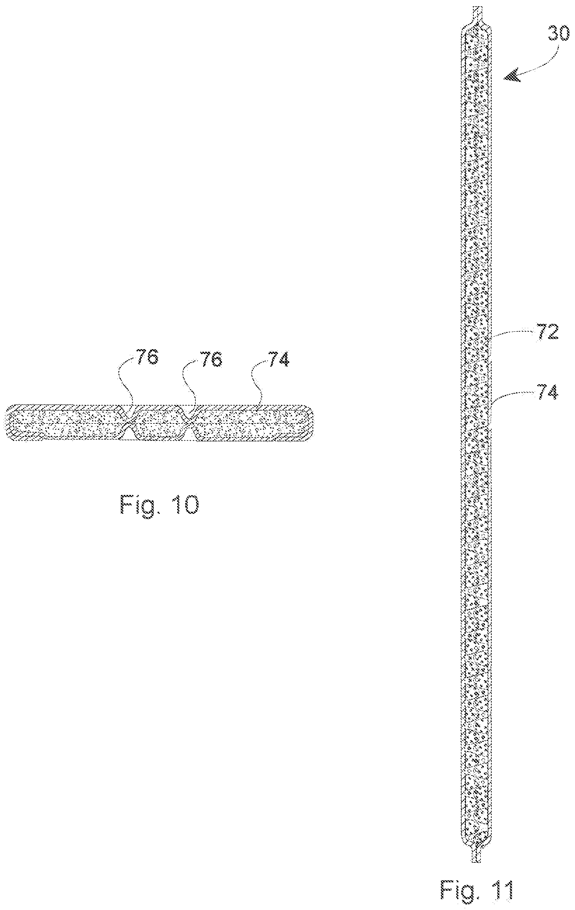

As used herein, the term "absorbent core" 30 refers to the component of the absorbent article 10 having the most absorbent capacity and that comprises an absorbent material. Referring to Figs. 9-11, in some instances, absorbent material 72 may be positioned within a core bag or a core wrap 74. The absorbent material may be profiled or not profiled, depending on the specific absorbent article. The absorbent core 30 may comprise, consist essentially of, or consist of, a core wrap, absorbent material 72, and glue enclosed within the core wrap. The absorbent material may comprise superabsorbent polymers, a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. An absorbent core periphery having a generally "dog bone" or "hour-glass" shape may taper along its width towards the crotch region 14 of the absorbent article 10.

Referring to Figs. 9-11, the absorbent core 30 may have areas having little or no absorbent material 72, where a wearer-facing surface of the core bag 74 may be joined to a garment-facing surface of the core bag 74. These areas having little or no absorbent material and may be referred to as "channels" 76. These channels can embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

### Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. **In** some instances, an elastic waistband may fully surround the waist opening circumference of an absorbent article. Nonwovens of the waistband may comprise a pattern or patterns of apertures and/or embossments and may be positioned over one or more graphics on a backsheet to achieve one or more of the benefits of the present disclosure.

### Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

The landing zone may comprise a pattern or patterns of apertures and/or embossments and may be positioned over the one or more graphics of the backsheet to achieve one or more of the benefits of the present disclosure. In some instances, the landing zones may be integral with a portion of the outer cover nonwoven materials. In other instances, the landing zones may be a discrete component or components.

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing surface of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78. At least a portion of the wetness indicator may be formed by one or more of the graphics disclosed herein.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2.

### Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. **In** other instances, a receiver may not be provided, but instead the condition of the absorbent article 10 may be visually or audibly apparent from the sensor on the absorbent article.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate number of absorbent articles per package. By packaging the absorbent articles under compression, caregivers can easily handle and store the packages, while also providing distribution savings to manufacturers owing to the size of the packages.

### Sanitary Napkin

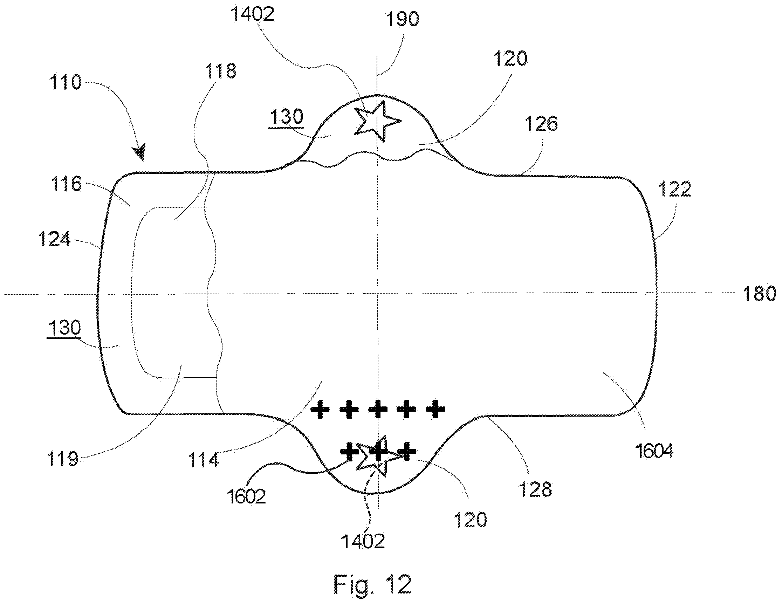

Referring to Fig. 12, an absorbent article of the present disclosure may be a sanitary napkin 110. The sanitary napkin 110 comprises a liquid permeable topsheet 114, a liquid impermeable backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

A sanitary napkin may comprise a topsheet comprising a pattern or patterns of apertures and/or embossments positioned over one or more graphics disposed on a backsheet to achieve one or more of the benefits of the present disclosure. In an instance, overlap of the pattern and the one or more graphics may occur in the wings. Since an absorbent core or secondary topsheet may not be present in the wings, perception of the topsheet pattern in the wings may be improved.

### Bio-Based Content for Absorbent Article Components

Components of the disposable absorbent article (i.e., diaper, disposable pant, adult incontinence article, sanitary napkin, pantiliner, etc.) described in this specification may at least partially be comprised of bio-sourced content as described in U.S. Pat. Appl. Publ. No. 2007/0219521 A1 Hird et al., published on September 20, 2007, U.S. Pat. Appl. Publ. No. 2011/0139658 A1 Hird et al., published on June 16, 2011, U.S. Pat. Appl. Publ. No. 2011/0139657 A1 Hird et al., published on June 16, 2011, U.S. Pat. Appl. Publ. No 2011/0152812 A1 Hird et al., published on June 23, 2011, U.S. Pat. Appl. Publ. No. 2011/0139662 A1 Hird et al., published on June 16, 2011, and U.S. Pat. Appl. Publ. No. 2011/0139659 A1 Hird et al., published on June 16, 2011. These components include, but are not limited to, topsheet nonwovens, backsheet films, backsheet nonwovens, side panel nonwovens, barrier leg cuff nonwovens, super absorbents, nonwoven acquisition layers, core wrap nonwovens, adhesives, fastener hooks, and fastener landing zone nonwovens and film bases.

In some forms, a disposable absorbent article component comprises a bio-based content value from about 10% to about 100% using ASTM D6866-10, method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. In a form, the disposable absorbent article component may be ground into particulates less than about 20 mesh using known grinding methods (e.g., Wiley^{®} mill), and a representative sample of suitable mass taken from the randomly mixed particles.

### Laminates

The laminates of the present disclosure comprise an outer cover material or a landing zone as discussed herein comprising a pattern and a backsheet comprising one or more graphics disposed on the garment-facing surface and/or the wearer-facing surface thereof. The one or more graphics disposed on the backsheet greatly increases the perception of the pattern formed in the outer cover material, the landing zone, and/or topsheet when the outer cover material and/or topsheet overlaps with the one or more graphics. The laminates may also comprise other materials. A portion of, or all of, the pattern of the outer cover material or landing zone, overlap a portion of, or all of, the one or more graphics. One example laminate is an outer cover material positioned over a backsheet film having one more graphics printed on a garment-facing surface of the backsheet. Another example - not encompassed by the claims - is a topsheet positioned over a backsheet film having one or more graphics printed on a wearer-facing surface of the backsheet. In some instances, the topsheet may overlap the one or more graphics of the backsheet film at least in wings of a sanitary napkin to form the laminate. In yet another example, a laminate is a landing zone positioned over a backsheet film having one or more graphics printed on a garment-facing surface of the backsheet. The landing zones may be integral with a portion of the outer cover nonwoven materials.

As will be discussed further below, the pattern of apertures and/or embossments of the outer cover material or the landing zone, and/or topsheet may form a recognizable, discrete indicia. The pattern of the outer-cover material, the landing zone, and/or topsheet and the one or more graphics of the backsheet may be sized such that an area of an individual recognizable, discrete indicium may be smaller than an area of the one or more graphics of the backsheet. In such a case, an entire area of at least one recognizable, discrete indicium may be overlapped by the portion of the one or more graphics, allowing for the individual recognizable, discrete indicium to be better perceived as the entire indicia is highlighted by the one or more graphics. The pattern of the outer-cover material, the landing zone, and/or topsheet and the one or more graphics of the backsheet may be sized such that an area of an individual recognizable, discrete indicium may be larger than, or the same as, an area of the one or more graphics of the backsheet.

### Backsheet Graphics

The backsheet of the laminate comprises one or more graphics. As used herein, the term "graphic" or "graphics" refers to a colored area of the backsheet. Color may be realized on the backsheet by dying the backsheet or a portion thereof, by applying an ink on the surface of the backsheet, for example, by printing on a surface of the backsheet, by incorporating a pigment into the backsheet or portions thereof, or by any other method of applying a color known in the art. A "graphic" may include the depiction of a design or designs, any recognizable indicia such as a number, a letter, a word, a brand name, an icon, a logo, a character, a portion of, or all of, a wetness indicator, a front/back indicator, any shape and/or symbol (for example hearts, clouds, animals, etc.), as well as a full flood of pigment across a surface, or a portion of a surface, of the backsheet. "Graphics" may also comprise visible placement indicia to indicate where a sensor should be attached to the absorbent article, such as a dashed outline that matches the shape of a sensor, for example.

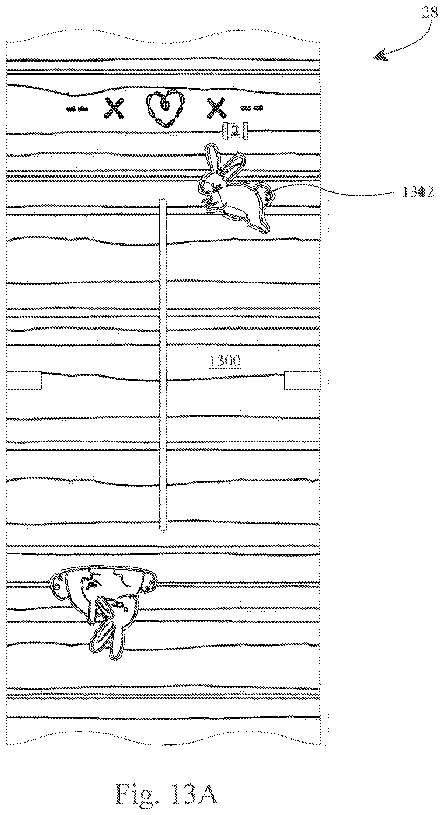

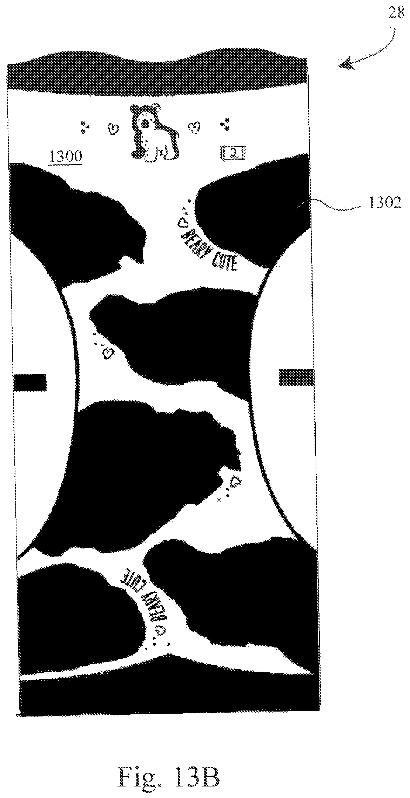

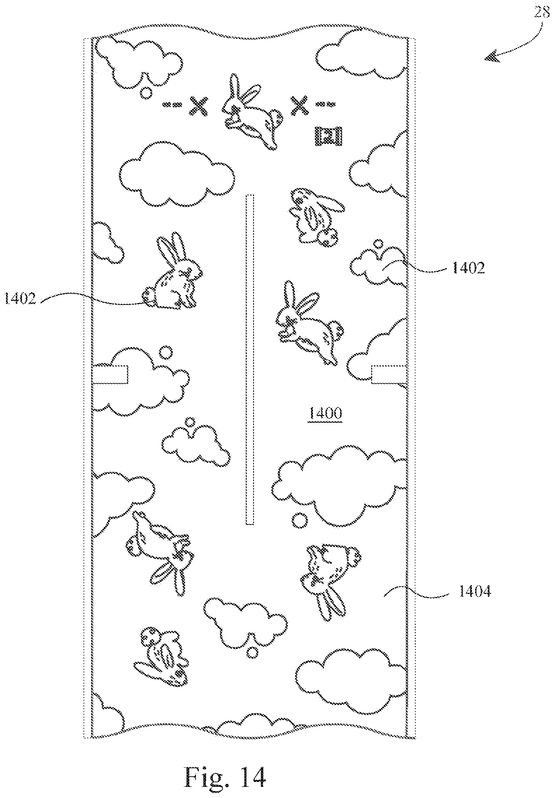

The one or more graphics are positioned on a a garment-facing surface of a backsheet. Figs. 13A and 13B are plan views of backsheets 28 comprising one or more graphics 1302 on a garment-facing surface 1300 and with the garment-facing surface 1300 facing the viewer. Fig. 14 is a plan view of a backsheet comprising one or more graphics 1402 on with a wearer-facing surface and with the wearer-facing surface 1400 facing the viewer. The absorbent article may be a diaper, a training pant, or an adult incontinence article. In another example, as shown in Fig. 12, the absorbent article is a sanitary napkin, and the wearer-facing surface 130 and/or the garment-facing surface of the backsheet 116 comprises one or more graphics 1402. The one or more graphics 1402 may be positioned on the backsheet 116 at any location, such as in the wings 120, for example. The one or more graphics 1402 may also be positioned in other locations of the backsheet 116.

The one or more graphics of the backsheet may comprise any one color or any combination of colors. The color and/or colors may be characterized by chroma value, according to the Backsheet Graphic Color Test Method described herein. Chroma values are a measure of the vividness or vibrancy of color in a backsheet graphic. The color and/or colors may be characterized by L*, a*, b* values, according to the Backsheet Graphic Color Test described herein. The L*, a*, b* color scale utilizes measures of lightness (L*), redness-greenness (a*), and yellowness-blueness (b*) to characterize colors. The color and/or colors may also be characterized by the PANTONE color system. The PANTONE color system uses a color numbering system to identify and match colors.

The one or more graphics of the backsheet may comprise a visually vibrant color or colors. The one or more graphics comprising a visually vibrant color or colors may be characterized by exhibiting a specific range of chroma values, L*, a*, b* values, and/or PANTONE values. A portion of the one or more graphics comprising the visually vibrant color or colors may exhibit a chroma value in the range of about 8 to about 130, about 8 to about 100, about 8 to about 90, about 8 to about 80, about 10 to about 95, about 10 to about 90, about 15 to about 80, about 20 to about 75, about 10 to about 60, about 10 to about 50, about 10 to about 45, about 19 to about 45, or about 13 to about 45, according to the Backsheet Graphic Color Test Method described herein, and specifically reciting all 0.5 increments within the specified ranges and all ranges formed therein or thereby. The portion of the one or more graphics may exhibit an L* value of less than 95, between about 0 and about 95, between about 5 and about 95, between about 8 and about 95, between about 15 and about 95, between about 25 and about 95, between about 40 and about 95, between about 50 and about 95, between about 60 and about 95, or between about 65 and about 95, according to the Backsheet Graphic Color Test Method described herein, and specifically reciting all 0.5 increments within the specified ranges and all ranges formed therein or thereby. A backsheet comprising one or more graphics comprising a visually vibrant color or colors may allow a pattern, or a portion of a pattern, of an overlying outer cover material and/or topsheet to stand out more prominently, as discussed further herein. A backsheet comprising one or more graphics comprising a visually vibrant color or colors may also provide overall vibrant backsheet graphics, even when overlaid with an outer cover material and/or topsheet.

Graphics comprising visually vibrant color characterized by the PANTONE color system herein may have an L* value in the range of about 5 to about 95, or the L* value maybe less than 95 or less than 90. An a* value may be in the range of about -90 to about 90, and a b* value may be in the range of about -90 to about 90. All L*, a*, and b* values are according to the Backsheet Graphic Color Test Method described herein, and specifically reciting all 0.5 increments within the specified ranges and all ranges formed therein or thereby.

Graphics that are a PANTONE color of 109 may have a chroma value in the range of about 5 to about 90, about 8 to about 90, about 18 to about 90, about 18 to about 84, about 27 to about 77, about 37 to about 66, or about 47 to about 56, according the Backsheet Graphic Color Test Method described herein, and specifically reciting all 0.5 increments within the specified ranges and all ranges formed therein or thereby. Graphics that are a PANTONE color of 109 may have an L* value in the range of about 86 to about 96, an a* value of about 0 to about 5, and a b* value of about 18 to about 84, according the Backsheet Graphic Color Test Method described herein, and specifically reciting all 0.5 increments within the specified ranges and all ranges formed therein or thereby.

Graphics that are a PANTONE color of 171 may have a chroma value in the range of about 15 to about 80, about 19 to about 75, about 26 to about 65, about 34 to about 57, or about 42 to about 49, according the Backsheet Graphic Color Test Method described herein, and specifically reciting all 0.5 increments within the specified ranges and all ranges formed therein or thereby. Graphics that are a PANTONE color of 171 may have an L* value in the range of about 60 to about 86, an a* value of about 15 to about 56, and a b* value of about 13 to about 48, according the Backsheet Graphic Color Test Method described herein, and specifically reciting all 0.5 increments within the specified ranges and all ranges formed therein or thereby.

Graphics that are a PANTONE color of 2965 may have a chroma value in the range of about 5 to about 30, about 7 to about 25, about 7 to about 21, about 8 to about 20, about 11 to about 18, about 13 to about 17, or about 13 to about 15, according the Backsheet Graphic Color Test Method described herein, and specifically reciting all 0.5 increments within the specified ranges and all ranges formed therein or thereby. Graphics that are a PANTONE color of 2965 may have an L* value in the range of about 14 to about 82, an a* value of about -7 to about -2, and a b* value of about -19 to about -5, according the Backsheet Graphic Color Test Method described herein, and specifically reciting all 0.5 increments within the specified ranges and all ranges formed therein or thereby.

Graphics that are a PANTONE color of 3272 may have a chroma value in the range of about 10 to about 70, about 11 to about 65, about 18 to about 65, about 25 to about 55, about 30 to about 50, about 31 to about 46, or about 31 to about 40, according the Backsheet Graphic Color Test Method described herein, and specifically reciting all 0.5 increments within the specified ranges and all ranges formed therein or thereby. Graphics that are a PANTONE color of 3272 may have an L* value in the range of about 58 to about 89, an a* value of about -60 to about -12, and a b* value of about -11 to about -3, according the Backsheet Graphic Color Test Method described herein, and specifically reciting all 0.5 increments within the specified ranges and all ranges formed therein or thereby.

Graphics that are a PANTONE color of 423 may have a chroma value in the range of about 1 to about 5, about 1 to about 3, about 1 to about 2.5, about 1 to about 2.2, about 1 to about 2, about 1 to about 1.5, about 1 to about 1.4, or about 1.4, according the Backsheet Graphic Color Test Method described herein, and specifically reciting all 0.5 increments within the specified ranges and all ranges formed therein or thereby. Graphics that are a PANTONE color of 423 may have an L* value in the range of about 58 to about 91, an a* value of about -2 to about -1, and a b* value of about -1 to about 0, according the Backsheet Graphic Color Test Method described herein, and specifically reciting all 0.5 increments within the specified ranges and all ranges formed therein or thereby.

Referring again to Figs. 13A, 13B, and 14, the garment-facing surface 1300 and/or the wearer-facing surface 1400 of the backsheet 28 may comprise a graphic-free area 1304, 1404 of from about 5% to about 85%, relative to an entire area of the respective surface of the backsheet 28. As used herein, the term "graphic-free area" refers to the percent area of the garment-facing surface 1300 and/or a wearer-facing surface 1400 of the backsheet 28 that does not exhibit the one or more graphics 1302, 1402. In other words, the graphic-free area 1304, 1404 is the total area of the garment-facing surface 1300 and/or a wearer-facing surface 1400 of the backsheet 28 that does not comprise ink, tint, pigment, or any other component of the one or more graphics 1302, 1402.

### Patterned Outer Cover Material and Topsheet

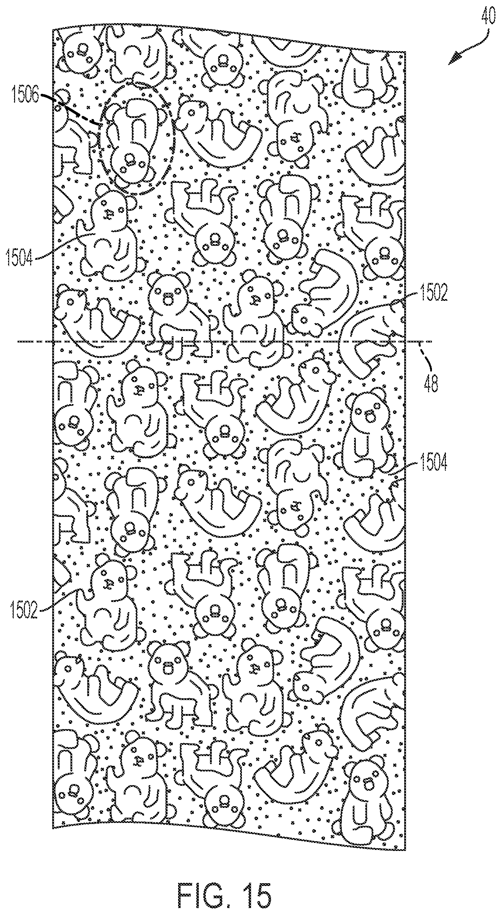

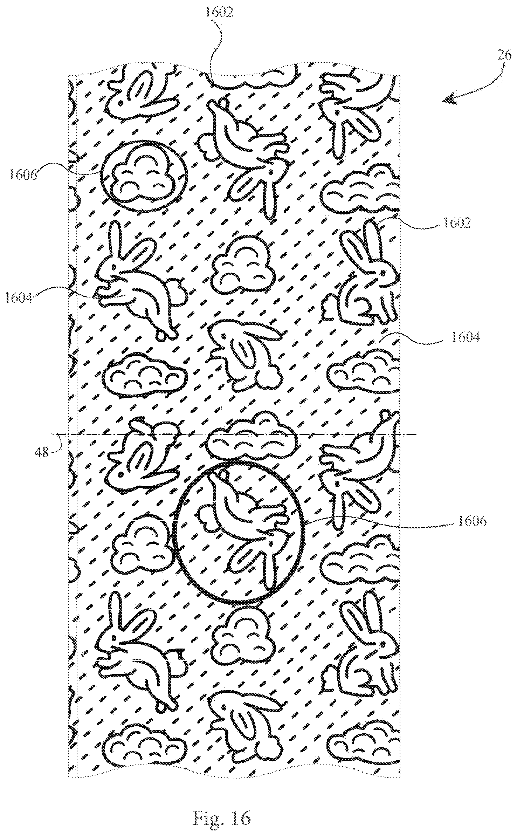

The outer cover material and/or the topsheet of the absorbent articles of the present disclosure may comprise a pattern or patterns. The pattern or patterns may be formed by, for example, embossing and/or aperturing the outer cover material and/or topsheet. The pattern may form at least 1 and less than 40, at least 5 and less than 25, at least 8 and less than 20, or at least 10 and less than 15 recognizable, discrete indicia in the outer cover material and/or the topsheet, and specifically reciting all integers within the specified ranges and all ranges formed therein or thereby. Recognizable, discrete indicia may be any recognizable indicia such as, for example, a number, a letter, an icon, a logo, any shape (for example hearts, clouds, animals, etc.), and groups of shapes that form one indicia (for example, a cluster of circles). For example, Fig. 15 show an outer cover material 40 comprising a pattern comprising a recognizable, discrete indicia 1506 in the shape of bears. Fig. 16 shows a topsheet 26 comprising a pattern comprising recognizable, discrete indicia 1606 in the shape of rabbits and clouds.

Recognizable, discrete indicia may be present in the outer cover material 40 and/or topsheet 26 in a size, scale, and disposition such that the indicia remains visually recognizable at a distance from the absorbent article. Referring to Figs. 15 and 16, any line taken parallel to a central lateral axis 48 across the outer cover material 40 and/or topsheet 26 may overlap between about 1 and about 15, between about 3 and about 12, or between about 5 and about 10 recognizable, discrete indicia, and specifically reciting all integers within the specified ranges and all ranges formed therein or thereby. A single recognizable, discrete indicium may have an area in the range of about 40 mm² to about 7,500 mm², about 45 mm² to about 7,400 mm², about 50 mm² to about 7,300 mm², or about 52 mm² to about 7,250 mm², and specifically reciting all 0.5 increments within the specified ranges and all ranges formed therein or thereby.

According to the present disclosure, the pattern of the outer cover material and/or topsheet may coordinate with and/or compliment the one or more graphics of the backsheet. For example, the wearer-facing surface and/or garment-facing surface of the backsheet may comprise one or more graphics in the form of a certain shape, design, and/or indicia, and an overlying outer cover material and/or topsheet may comprise a pattern in that same shape, design, and/or indicia. **In** one example, the one or more graphics may be in the shape of a heart or hearts, and the pattern in the outer cover material and/or topsheet may also be in the shape of a heart or hearts, and may overlap a portion of the backsheet. **In** another example, as shown in Fig. 16, a topsheet 28 or outer cover material may comprise a pattern, a portion of which may be in the shape of a rabbit, and may overlay a backsheet 28 comprising one or more graphics 1402 in the shape of a rabbit, as shown in Fig. 14.

### Embossed Pattern

The outer cover material 40 and/or the topsheet 26 may comprise an embossed pattern. As used herein, the term "emboss" or "embossed" refers to forming a pattern in a material by densification of a portion of the fibers of the material resulting in densified regions and non-densified regions. The non-densified regions may retain an amount of loft and stand out in relief to the densified regions. A pattern or patterns may be formed with respect to the densified regions and/or with respect to the non-densified regions.

Any process known in the industry for embossing continuous or discrete webs of material may be used to create embossing. Generally, such processes utilize a rotary process having an embossing roller comprising projections that form a pattern and a pressure roller. **In** such a process, a web of material may be fed through a nip defined between the embossing roller and the pressure roller, wherein the nip has a width less than the thickness of the material to be embossed. The pressure created by the projections contacting the pressure roller in the nip may form one or more densified regions in the web of material. The densified regions may take on the pattern from the projections of the embossing roller. Portions of the web of material that pass through the embossing roller without coming into contact with projections may be non-densified regions and may retain at least some of, or all of, their loft.

Referring again to Fig. 15, the outer cover material 40 may comprise an embossed pattern comprising at least one densified region 1502 and at least one non-densified region 1504. Referring to Fig. 16, the topsheet 26 may comprise an embossed pattern comprising at least one densified region 1602 and at least one non-densified region 1604. At least a portion of the densified regions 1502, 1602 and/or the non-densified regions 1504, 1604 may form a recognizable, discrete indicia 1506, 1606 in the outer cover material 40 and/or the topsheet 26, respectively. The topsheet 26 and/or the outer cover material 40 may comprise at least 1 and less than 40, at least 5 and less than 30, at least 8 and less than 25, or at least 10 and less than 20 recognizable, discrete indicia 1506, 1606, formed from at least a portion of the densified regions 1502, 1602 and/or the non-densified regions 1504, 1604, and specifically reciting all integers within the specified ranges and all ranges formed therein or thereby.

The outer cover material 40 and/or topsheet 26 may comprise a total densified region area in the range of about 5% to about 40%, about 10% to about 30%, about 10% to about 25%, relative to an entire area of the respective outer cover material 40 and/or topsheet 26.

A portion of the densified region of the embossed pattern of the outer cover material and/or topsheet may overlap at least a portion of the one or more graphics of the backsheet. The densified region may filter underlying colors differently as compared to the non-densified regions, leading to a visual color contrast between the portions of the densified and non-densified regions of the embossed pattern that overlap the graphic of the backsheet. Without wishing to be bound by theory, it is believed that increasing contrast (e.g., increasing Delta E) between portions of the densified and non-densified regions of the embossed pattern, especially when the embossed pattern comprises relatively large recognizable, discrete indicia, allows for improved visual perception of such indicia. **In** an instance, a portion of an embossed pattern of an outer cover material overlaps one or more backsheet graphics comprising a visually vibrant color. **In** another instance, a portion of an embossed pattern of a topsheet overlaps one or more backsheet graphics comprising a visually vibrant color.

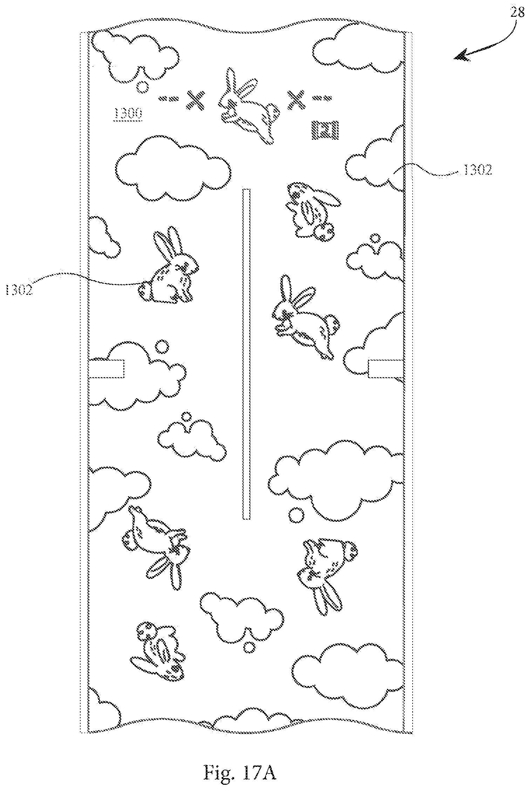

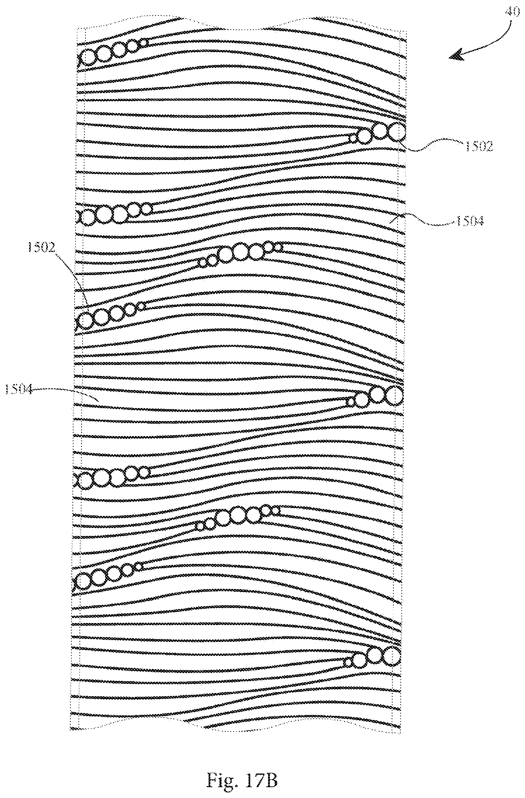

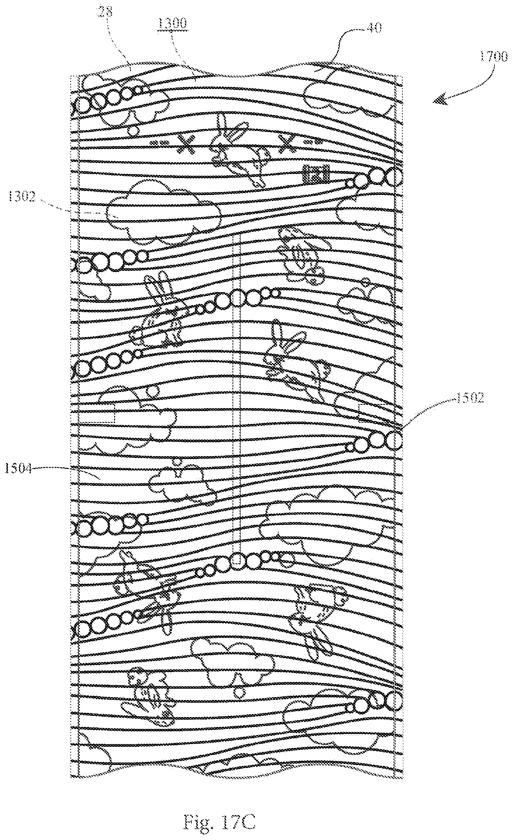

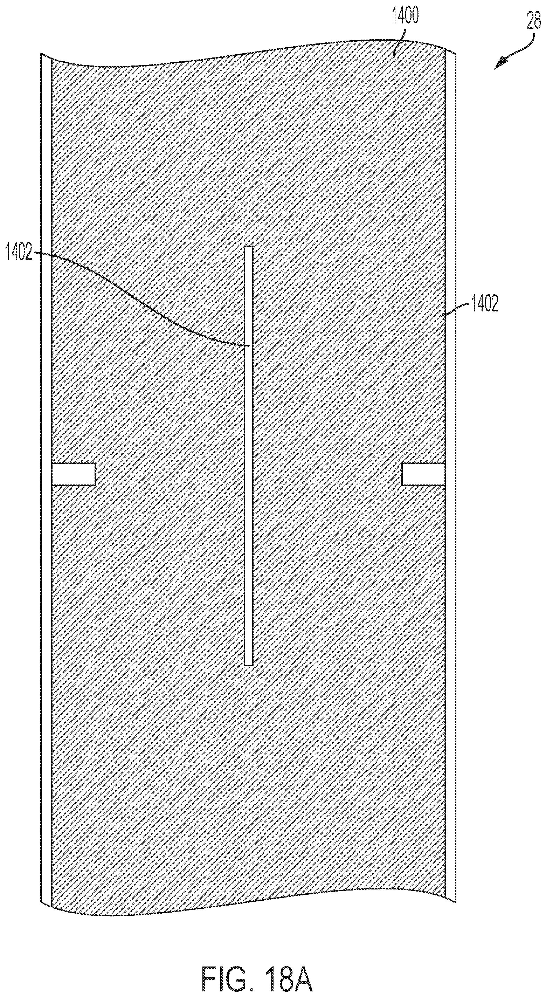

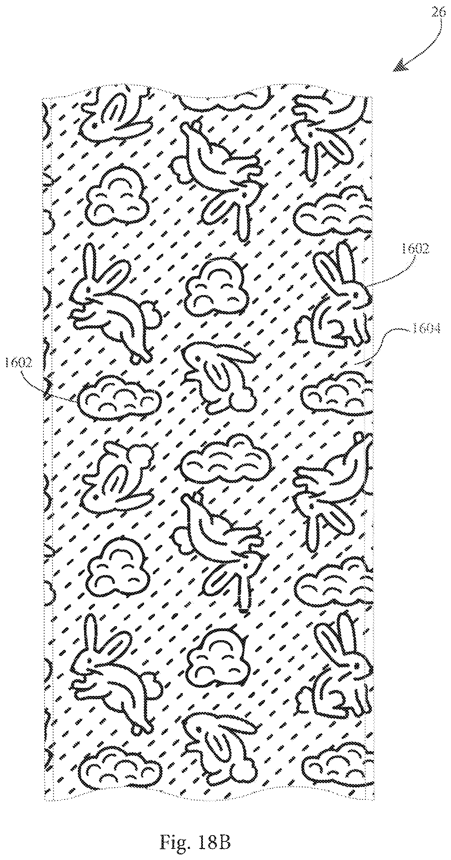

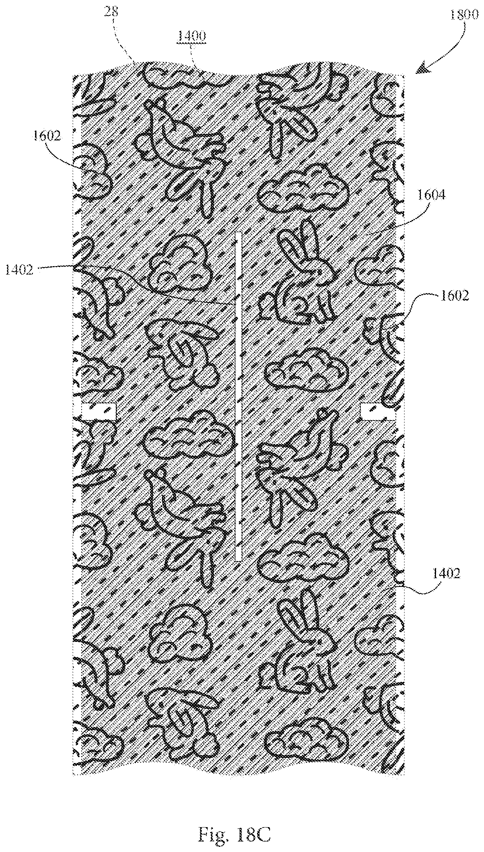

Fig. 17A is a plan view of a backsheet 28 comprising one or more graphics 1302, the garment-facing surface 1300 facing the viewer. Fig. 17B is a plan view of an outer cover material 40 comprising an embossed pattern comprising a densified region 1502 and a non-densified region 1504. Fig. 17C is a plan view of an outer cover-backsheet laminate 1700 comprising the outer cover material 40 of Fig. 17B overlaying the backsheet 28 of Fig. 17A. As shown in Fig. 17C, a portion of the densified region 1502 of the embossed pattern of the outer cover material 40 overlaps a portion of the one or more graphics 1302 of the garment-facing surface 1300 of the backsheet 28. Fig. 18A is a plan view of a backsheet 28 comprising one or more graphics 1402, the wearer-facing surface 1400 facing the viewer. Fig. 18B is a plan view of a topsheet 26 comprising an embossed pattern comprising at least one densified region 1602 and at least one non-densified region 1604. Fig. 18C is a plan view of a topsheet-backsheet laminate 1800 comprising the topsheet 26 of Fig. 18B overlaying the backsheet 28 of Fig. 18A. As shown in Fig. 18C, a portion of the densified region 1602 of the embossed pattern of the topsheet 26 overlaps a portion of the one or more graphics 1402 of the wearer-facing surface 1400 of the backsheet 28. Referring again to Fig. 12, the liquid permeable topsheet 114 of the sanitary napkin 110, or portions thereof, may comprise an embossed pattern comprising one or more densified regions 1602 and one or more non-densified regions 1604. A portion of, or all of, the densified region 1602 may overlap with a portion of, or all of, the one or more graphics, 1402. Disposition of a portion of the one or more densified regions over a portion of the one or more graphics may improve consumer perception of the embossed pattern comprising recognizable, discrete indicia. Areas of overlap between the one or more densified regions and portions of the one or more graphics may have a first color value, and areas of overlap between the one or more non-densified regions and portions of the one or more graphics may have a second, and different color value.

Regarding the outer cover-backsheet laminates of the present disclosure, a first portion of the one or more graphics exhibits a first L*, a*, b* color value, when measured through the densified regions of the embossed pattern of the outer cover material, according to the Backsheet Laminate Color Test Method described herein. A second portion of the one or more graphics exhibits a second, different L*, a*, b* color value, when measured through the non-densified regions of the outer cover material, according to the Backsheet Laminate Color Test Method described herein. The difference between the color perception of the one or more graphics as measured through the densified regions compared to the color perception of the one or more graphics measured through the non-densified regions may be characterized by calculating the delta E value, according to the Backsheet Laminate Color Test Method described herein. The delta E value between the first L*, a*, b* color value and the second L*, a*, b* color value may be at least 2, according to the Backsheet Laminate Color Test Method described herein. The delta E value between the first L*, a*, b* color value and the second L*, a*, b* color value may be between about 2 and about 50, between about 3.4 and about 40, between about 6 and about 35, or between about 10 and about 25, according to the Backsheet Laminate Color Test Method described herein, and specifically reciting all 0.1 increments within the specified ranges and all ranges formed therein or thereby. A delta E value greater than 2, or greater than 3.4, is perceptible by the human eye, and may highlight the embossed pattern in the outer cover material and/or topsheet when overlapped with the one or more graphics, thereby increasing perception of the pattern.

It is also believed that graphics, or portions of graphics, having particular color characteristics may improve the visual perception of embossed patterns when disposed under at least a portion of the one or more densified regions of the embossed pattern. Graphics comprising visually vibrant colors may resist muting and provide a vibrant graphic perception when viewed through the outer cover material and/or the topsheet. A portion of the one or more graphics may have a first L* color value in the range of about 0 to about 95, about 5 to about 93, or about 10 to about 90; a first a* color value in the range of about -128 to about 128 about -90 to about 90, about -75 to about 80, or about -65 to about 60; and a first b* color value in the range of about -120 to about 120, about -90 to about 100, about -50 to about 90, or about -25 to about 90, when measured through the one or more densified regions of the embossed pattern of the outer cover material and/or the topsheet, according to the Backsheet Laminate Color Test Method described herein, and specifically reciting all 1.0 increments within the specified ranges and all ranges formed therein or thereby. A portion of the one or more graphics may have a second L* color value in the range of about 0 to about 95, about 5 to about 93, or about 10 to about 90; a second a* color value in the range of about -128 to about 128 about -90 to about 90, about -75 to about 80, or about -65 to about 60; and a second b* color value in the range of about -120 to about 120, about -90 to about 100, about -50 to about 90, or about -25 to about 90, when measured through the one or more non-densified regions of the embossed pattern of the outer cover material and/or the topsheet according to the Backsheet Laminate Color Test Method described herein, and specifically reciting all 1.0 increments within the specified ranges and all ranges formed therein or thereby.

### Apertured Pattern

According to the present disclosure, the outer cover material and/or the topsheet may comprise a plurality of apertures. At least a portion of the plurality of apertures may form an apertured pattern. A portion of the plurality of apertures may be grouped in arrays of apertures. Apertures of an aperture array may be spaced such that a pattern emerges with respect to at least a portion of the apertures and/or with respect to at least a portion of the surrounding non-apertured areas in the outer cover material and/or topsheet.

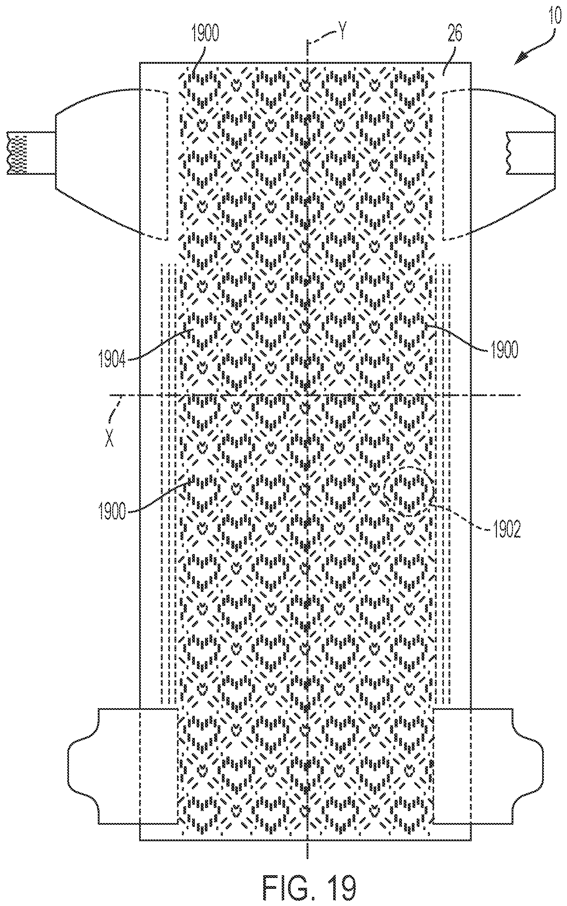

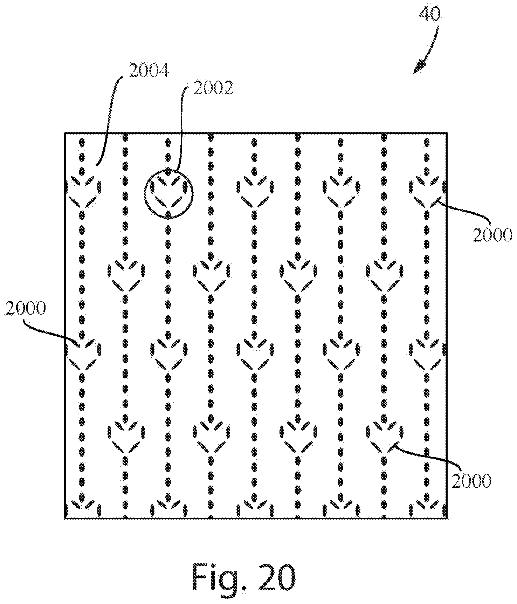

Referring to Fig. 19, the absorbent article 10 may comprise a topsheet 26 that comprises a plurality of apertures 1900 and a non-apertured region 1904. At least a portion of the plurality of apertures 1900 may form a recognizable, discrete indicia 1902 in the topsheet 26, such as hearts, for example. Referring to Fig. 20, the absorbent article may comprise an outer cover material 40 that comprises a plurality of apertures 2000 and non-apertured regions 2004. At least a portion of the plurality of apertures 2000 may form a recognizable, discrete indicia 2002 in the outer cover material 40. The topsheet 26 and/or the outer cover material 40 may comprise at least 1 and less than 40, at least 5 and less than 30, at least 8 and less than 25, or at least 10 and less than 20 recognizable, discrete indicia 1902, 2002, formed from at least a portion of the plurality of apertures 1900, 2000, and specifically reciting all integers within the specified ranges and all ranges formed therein or thereby.

The topsheet and/or outer cover material comprising a plurality of apertures may have an open area of between about 7% and about 30%, between about 10% and about 25%, or between about 12% and about 20%, specifically reciting all 1% increments within the specified ranges and all ranges formed therein or thereby, according to the Open Area Test described herein.

The plurality of apertures may be formed in the outer cover material and/or topsheet using any known method, including pin aperturing and water-jet punching, for example. Apertures may also be produced by a process of overbonding (intermittently densifying) the topsheet and/or outer cover precursor material prior to subjecting the material to a cross-machine directional strain, causing at least some of the overbonds to at least partially rupture, as disclosed in U.S. Pat. No. 5,628,097 to Benson et al., issued on May 13, 1997, and disclosed in U.S. Pat. Appl. Publication No. 2016/0136014 to Arora et al.

The absorbent articles of the present disclosure may comprise a laminate or laminates formed of a portion of the outer cover material and/or the topsheet comprising an apertured pattern and overlaying a portion of the backsheet that comprises one or more graphic. Like Figs. 17C and 18C, where the one or more densified regions of the embossed pattern overlapped the one or more graphics of the backsheet, a portion of the plurality of apertures of the outer cover material and/or the topsheet may overlap a portion of the one or more graphics of the backsheet. Overlap of the one or more graphics by a portion of the plurality of apertures may result in a visual color contrast between the plurality of apertures and the non-apertured portions of the outer cover material and/or topsheet in the overlap region. Without wishing to be bound by theory, it is believed that increasing contrast (e.g., Delta E) between a portion of the plurality of apertures and the non-apertured region of the outer cover material and/or topsheet, especially when the overlapping portion of the plurality of apertures forms a relatively large, recognizable, and discrete indicia, may result in improved visual perception of such indicia. In addition, where the one or more graphics of the backsheet comprises a visually vibrant color, as discussed herein, the pattern formed by at least a portion of the plurality of apertures may stand out more prominently as compared to backsheet graphics that comprise more muted colors. As such, areas of overlap between the outer cover material and/or topsheet and portions of the graphics of the backsheet in an outer-cover-backsheet and/or topsheet-backsheet laminate may have a first color value when measured through an aperture, and may have a second, different color value when measured through non-apertured areas.

Regarding the outer cover-backsheet laminates and/or the topsheet-backsheet laminates of the present disclosure, a first portion of the one or more graphics may exhibit a first L*, a*, b* color value, when measured through an aperture of the plurality of apertures of the outer cover material and/or the topsheet, according to the Backsheet Laminate Color Test Method described herein. A second portion of the one or more graphics may exhibit a second, different L*, a*, b* color value, when measured through the non-apertured region of the outer cover material and/or the topsheet, according to the Backsheet Laminate Color Test Method described herein. The difference between the color perception of the graphic as measured through the aperture compared to the color perception of the graphic measured through the non-apertured region may be characterized by calculating the delta E value according to the Backsheet Laminate Color Test Method described herein. The delta E value between the first L*, a*, b* color value and the second L*, a*, b* color value may be at least 2, according to the Backsheet Laminate Color Test Method described herein. The delta E value between the first L*, a*, b* color value and the second L*, a*, b* color value may be between about 2 and about 50, between about 3.4 and about 40, between about 6 and about 35, or between about 10 and about 25, according to the Backsheet Laminate Color Test Method described herein, and specifically reciting all 0.1 increments within the specified ranges and all ranges formed therein or thereby. A delta E value greater than 2, or greater than 3.4, is perceptible by the human eye, and may highlight the pattern formed by the plurality of apertures in the outer cover material and/or topsheet, thereby increasing perception of the pattern and/or indicia.

It is believed that graphics, or portions of graphics, having particular color characteristics may improve the visual perception of apertured patterns when such a graphic is disposed under at least a portion of the plurality of apertures of the outer cover and/or topsheet. The presence of the plurality of apertures in the outer cover material and/or topsheet may improve the perception of visually vibrant graphics, or graphics comprising visually vibrant colors, when viewed through the outer cover material and/or the topsheet. A portion of the one or more graphics may have a first L* color value in the range of about 0 to about 95, about 5 to about 93, or about 10 to about 90; a first a* color value in the range of about -128 to about 128 about -90 to about 90, about -75 to about 80, or about -65 to about 60; and a first b* color value in the range of about -120 to about 120, about -90 to about 100, about -50 to about 90, or about -25 to about 90, when measured through an aperture of the plurality of apertures of the outer cover material and/or the topsheet, according to the Backsheet Laminate Color Test Method described herein, and specifically reciting all integers within the specified ranges and all ranges formed therein or thereby. A portion of the one or more graphics may have a second L* color value in the range of about 0 to about 95, about 5 to about 93, or about 10 to about 90; a second a* color value in the range of about -128 to about 128 about -90 to about 90, about -75 to about 80, or about -65 to about 60; and a second b* color value in the range of about -120 to about 120, about -90 to about 100, about -50 to about 90, or about -25 to about 90, when measured through the non-apertured region of the outer cover material and/or the topsheet, according to the Backsheet Laminate Color Test Method described herein, and specifically reciting all integers within the specified ranges and all ranges formed therein or thereby.

### Stitch-like Patterns

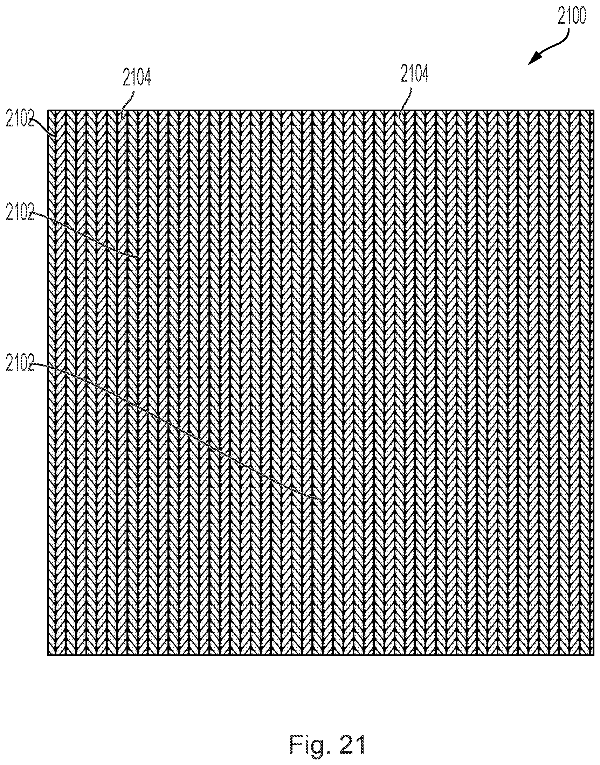

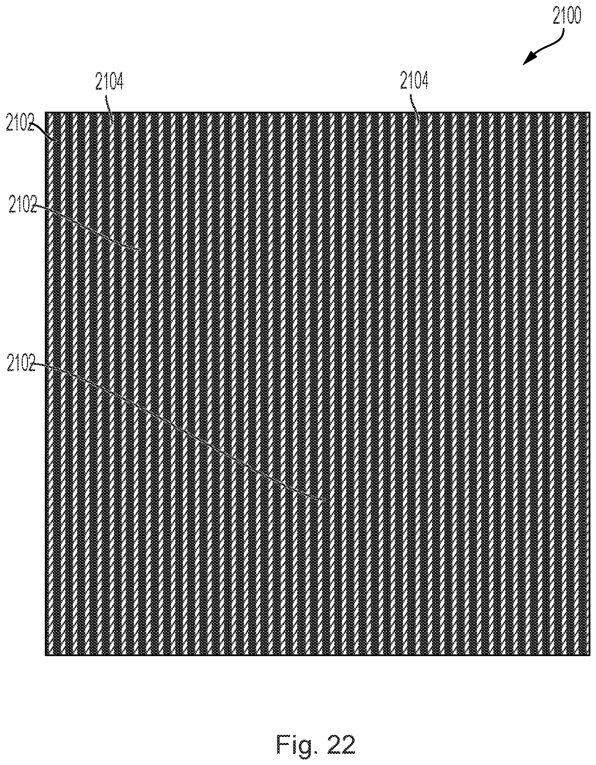

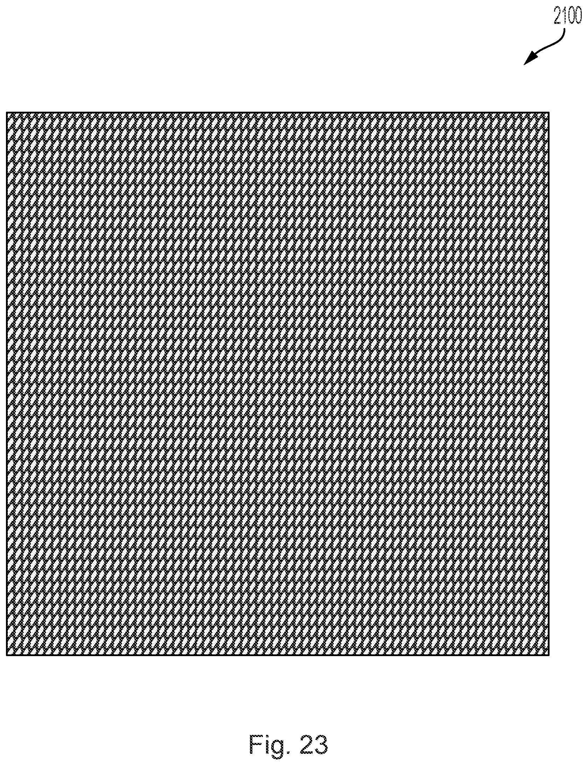

The backsheet, in addition to the various graphics discussed herein, may comprise a stitch-like pattern on the garment-facing surface or the wearer-facing surface thereof. The stitch-like pattern may surround, or partially surround the graphics. Typically, the stitch-like pattern does not overlap the graphics, or is free from overlap with the graphics. In some instances, the stitch-like pattern may overlap the graphics or portions thereof. In some instances, a stitch-like pattern may be used without the graphics. Figs. 21-23 illustrate examples of stitch-like patterns 2100. Of course, graphics may also be present within the stitch-like patterns. The stitch-like patterns on backsheets paired with the outer cover materials, the landing zones, and/or the topsheets with embossed and/or apertured patterns disclosed herein provide the absorbent articles of the present disclosure with a clothing-like, soft appearance, which is consumer desirable. As depicted in Figs. 21 and 22, the stitch-like patterns may comprise a plurality of linear elements 2102. In an absorbent article, the linear elements 2102 typically extend in a direction generally parallel to the central longitudinal axis of the absorbent article, but may also extend in other directions. Generally parallel, in this context, means +/- 10 degrees relative to the central longitudinal axis of the absorbent article. The linear elements may also extend in a range of about 11 degrees to about 90 degrees relative to the central longitudinal axis of the absorbent article, such as about 45 degrees, for example. The linear elements may be continuous or discontinuous. The linear elements may contain arcuate portions so as to form wavy linear elements. The linear elements may be spaced a distance apart from each other in a horizontal direction. That distance may be in the range of about 1mm to about 15mm, about 1.5mm to about 15mm, about 2mm to about 12mm, about 2mm to about 10mm, about 2mm to about 8mm, about 2mm to about Smm, or about 1.5mm to about 4mm, specifically reciting all 0.1mm increments within the specified ranges and all ranges formed therein or thereby. The distance between two adjacent linear elements may vary or may be consistent within a stitch-like pattern. Distances below 1mm between the linear elements create the impression of a "flood" of color and significantly reduce the aesthetic benefits of the stitch-like pattern. The linear elements themselves may have a horizontal thickness in the range of about 0.2mm to about 5mm, about 0.3mm to about 5mm, about 0.4mm to about 4mm, or about 0.5 to about 3mm, specifically reciting all 0.1mm increments within the specified ranges and all ranges formed therein or thereby. Two adjacent linear elements 2102 may be connected to each other by a plurality of connecting elements 2104. The connecting elements 2104 may extend generally horizontally between the liner elements 2102 or may extend at non-horizontal angle. Different connecting elements may extend in the same direction or in different directions. From about 5 to about 100 connecting elements 2104 may extend between two adjacent linear elements 2102. In other stitch-like patterns, such as the pattern illustrated in Fig. 23, no linear elements may be provided. The stitch-like pattern may cover about 20% to about 95% of an overall surface area of the backsheet.

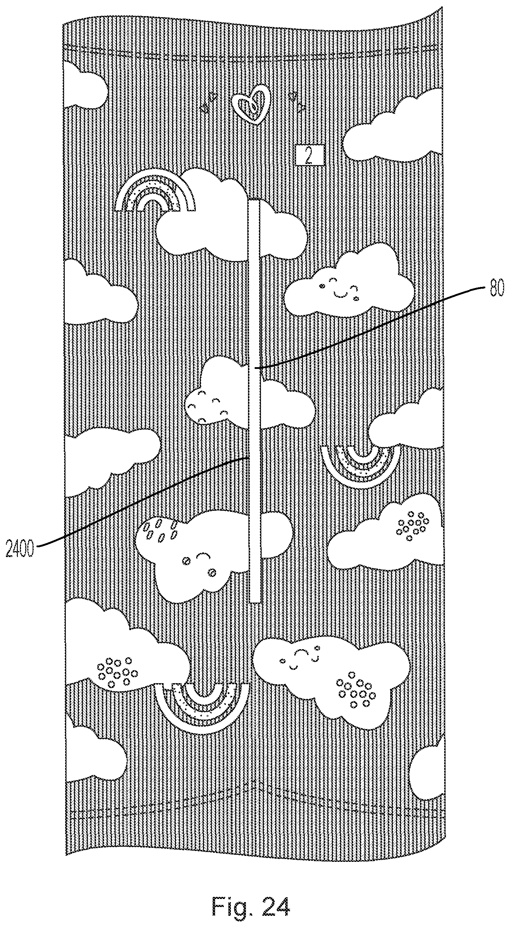

Fig. 24 is a plan view of a liquid impermeable backsheet comprising one or more visually vibrant graphics. The wetness indicator 80 may or may not comprise a portion of the one or more visually vibrant graphics. The backsheet may comprise a stitch-like pattern as described above. In some instances, it may be desirable to include a non-printed zone, a no graphics zone, a light graphics zone, a no color zone, or a white zone (together "zone 2400") surrounding the wetness indicator 80 so that the wetness indicator 80 remains clearly visible to a caregiver. In other instances, the zone 2400 surrounding the wetness indicator 80 may be a different color than at least a portion of the remainder of the graphics and the wetness indicator so that the wetness indicator remains clearly visible to a caregiver. In an instance, the zone 2400 may be phased or have a gradient to make the wetness indicator 80 more visible and have less of a harsh line of graphics/no graphics or color/no color. For instance, graphics in the zone 2400 more distal from the wetness indicator 80 may be darker or more visible than graphics in the zone 2400 more proximal to the wetness indicator 80. In another instance, graphics in the zone 2400 more distal from the wetness indicator 80 may be visible while areas of the zone 2400 more proximal to the wetness indicator may be graphics free or color free. The zone 2400 of different color or graphics or the zone of no color or graphics may fully, or at least partially, surround the wetness indicator 80.

### Test Methods:

### Backsheet Graphic Color Test Method

Chroma values are a measure of the vividness or vibrancy of color in a backsheet graphic. Generally, Chroma is calculated from the reflectance measurements of the CIE 1976 L*a*b* color values. Chroma is measured using a spectrophotometer with a computer interface (a suitable instrument is the HunterLab LabScan XE running Universal Software, as available from Hunter Associates Laboratory Inc., Reston, VA). All testing is performed in a conditioned room maintained at about 23 ± 2 °C and about 50 ± 2 % relative humidity.

To obtain a sample, carefully remove the backsheet film layer of material from an absorbent article. A cryogenic spray (such as Cyto-Freeze, Control Company, Houston TX) can be used to remove the sample from the underlying and overlaying layers if necessary. Identify a region of the sample containing a homogeneous colored graphic as the testing site. If the sample at the testing site contains any holes, tears, or other physical deformations another site is to be selected. Ensure that all adhesive and nonwoven fibers have been completely removed from the testing site. A layer of raw backsheet film material with a graphic, obtained prior to incorporation into an absorbent article, may also be tested. Precondition the samples at about 23 °C ± 2 C° and about 50% ± 2% relative humidity for 2 hours prior to testing.

Select the disk with the largest measurement port size that can fit within the selected homogeneous colored graphic testing site. Standardize the instrument using the selected port size (indicate the appropriate area view to the software) utilizing the manufacturer supplied black tile, then white tile. Calibrate the instrument according to manufacturer's specifications using their supplied standard tiles. Configure the software to measure color using the CIE 1976 L*a*b* color scale, D65 illuminant and 10° standard observer.

Place the sample over the measurement port, with the garment-facing surface or the wearer-facing surface oriented toward the instrument, depending on what surface the graphic is on. Gently pull the sample taut, without stretching, to ensure that it does not pillow into the port, and then back it with the standard white tile. Make sure that the area of the sample to be measured faces the port and completely covers the port. Take a reading and record the individual L*, a*, and b* values, then remove the white tile and sample. The individual L*, a*, and b* values are recorded to the nearest 0.1 units.

Calculate the Chroma value for the sample according to the following equation: $Chroma = \sqrt{{a}^{∗ 2} + {b}^{∗ 2}}$ The Chroma value for the sample is recorded to the nearest 0.1 units.

### Backsheet Laminate Color Test Method

The purpose of this test is to measure the CIE 1976 L*a*b* color values of a backsheet graphic visible through a region and/or aperture of an outer cover material, landing zone, and/or topsheet, and calculate the delta E, or the magnitude of color difference, between two regions. A flatbed scanner capable of scanning a minimum of 24 bit color at 2400 dpi with manual control of color management (a suitable scanner is an Epson Perfection V750 Pro from Epson America Inc., Long Beach CA, or equivalent) is used to acquire images. The scanner is interfaced with a computer running color calibration software capable of calibrating the scanner against a color reflection IT8 target utilizing a corresponding reference file compliant with ANSI method IT8.7/2-1993 (suitable color calibration software is Monaco EZColor or i1Studio available from X-Rite Grand Rapids, MI, or equivalent). The color calibration software constructs an International Color Consortium (ICC) color profile for the scanner, which is used to color correct an output image using an image analysis program that supports application of ICC profiles (a suitable program is Photoshop available from Adobe Systems Inc., San Jose, CA, or equivalent). The color corrected image is then converted to into the CIE L*a*b* color space for subsequent color analysis (a suitable image color analysis software is MATLAB available from The Mathworks, Inc., Natick, MA).

To obtain a sample, carefully remove the outer cover-backsheet laminate, landing zone-backsheet laminate, or topsheet-backsheet laminate from an absorbent article. A cryogenic spray (such as Cyto-Freeze, Control Company, Houston TX) can be used to remove the sample from the underlying layers if necessary. Identify a portion of the sample where the densified region of the embossed pattern or an aperture in the outer cover material, landing zone, or topsheet overlaps a portion of a homogeneous colored graphic on the backsheet as the testing site. If the sample at the testing site contains any holes, tears, or other physical deformations, other than the aperture or densified region of the embossed pattern, another site is to be selected. An outer cover-backsheet **laminate,** landing zone-backsheet laminate, and/or a topsheet-backsheet laminate with a backsheet comprising one or more graphics obtained prior to incorporation into an absorbent article may also be tested. Precondition the samples at about 23 °C ± 2 C° and about 50% ± 2% relative humidity for 2 hours prior to testing.

The scanner is turned on 30 minutes prior to calibration and image acquisition. Deselect any automatic color correction or color management options that may be included in the scanner software. If the automatic color management cannot be disabled, the scanner is not appropriate for this application. The recommended procedures of the color calibration software are followed to create and export an ICC color profile for the scanner. The color calibration software compares an acquired IT8 target image to a corresponding reference file to create and export the ICC color profile for a scanner, which will be applied within the image analysis program to correct the color of subsequent output images.

The scanner lid is opened and the sample carefully laid flat on the center of the scanner glass with the embossed and/or apertured surface of the testing site oriented toward the glass. A scan containing the entire testing site is acquired and imported into the image analysis software at 24 bit color with a resolution of 2400 dpi (approximately 94.5 pixels per mm) in reflectance mode. The ICC color profile is assigned to the image producing a color corrected sRGB image. This calibrated image is saved in an uncompressed format to retain the calibrated R,G,B color values, such as a TIFF file, prior to analysis.

The sRGB color calibrated image is opened in the color analysis software, and converted into the CIE L*a*b* color space. This is accomplished by the following procedure. First, the sRGB data is scaled into a range of [0, 1] by dividing each of the values by 255. Then the companded sRGB channels (denoted with upper case (R,G,B), or generically V) are linearized (denoted with lower case (r,g,b), or generically v) as the following operation is performed on all three channels (R, G, and B): $V ∈ \left\{R, G, B\right\}$ $v ∈ \left\{r, g, b\right\}$ $v = \left\{\begin{matrix}\frac{V}{12.92} if V \leq 0.04045 \\ {\left(\frac{V + 0.055}{1.055}\right)}^{2.4} otherwise\end{matrix}\right\}$ The linear r, g, and b values are then multiplied by a matrix to obtain the XYZ Tristimulus values according to the following formula: $\left[\begin{matrix}X \\ Y \\ Z\end{matrix}\right] = \left[\begin{matrix}0.4124 & 0.3576 & 0.1805 \\ 0.2126 & 0.7152 & 0.0722 \\ 0.0193 & 0.1192 & 0.9505\end{matrix}\right] \left[\begin{matrix}r \\ g \\ b\end{matrix}\right]$

The XYZ Tristimulus values are rescaled by multiplying the values by 100, and then converted into CIE 1976 L*a*b* values as defined in CIE 15:2004 section 8.2.1.1 using D65 reference white.

The CIE L*a*b* image is analyzed by identifying and manually drawing a region of interest (ROI) around the densified region or aperture opening of a first region. The average L*, a*, and b* color values within the ROI are measured and recorded as L*₁, a*₁, and b*₁. A second non-densified and/or non-apertuerd region is identified and a region of interest (ROI) is manually drawn around its perimeter. The average L*, a*, and b* color values are then measured for the second region, and recorded as L*₂, a*₂, and b*₂. The Delta E value is then calculated according to the following equation: $Delta E = \sqrt{{\left({L}_{2}^{∗}-{L}_{1}^{∗}\right)}^{2} + {\left({a}_{2}^{∗}-{a}_{1}^{∗}\right)}^{2} + {\left({b}_{2}^{∗}-{b}_{1}^{∗}\right)}^{2}}$

The individual L*, a*, and b* color values for the two regions, and Delta E value are reported to the nearest 0.1 units.

### Open Area Test Method

The percent open area may be obtained from aperture specimen images acquired using a flatbed scanner. The scanner is capable of scanning in reflectance mode at a resolution of 6400 dpi and 8 bit grayscale (a suitable scanner is an Epson Perfection V750 Pro from Epson America Inc., Long Beach CA, or equivalent). The scanner is interfaced with a computer running an image analysis program (a suitable program is ImageJ v. 1.47, National Institute of Health, USA, or equivalent). The specimen images are distance calibrated against an acquired image of a ruler certified by NIST. The aperture specimen is backed with a black glass tile (P/N 11-0050-30, available from HunterLab, Reston, VA, or equivalent) prior to acquiring the image. The resulting grayscale image is then converted to a binary image via a threshold gray-level value, enabling the separation of open aperture regions from specimen material regions, and these regions analyzed using the image analysis program. All testing is performed in a conditioned room maintained at about 23 ± 2 °C and about 50 ± 2 % relative humidity.

### Sample Preparation:

To obtain a specimen, the absorbent article or other product is taped to a rigid flat surface in a planar configuration. Any leg elastics present may be cut to facilitate laying the article flat. The outer boundary of the region lying above the absorbent core of the article is identified and marked on the apertured layer. The specimen of apertured layer is removed from the underlying layers of the article by cutting around the outer perimeter of the article with a razor blade. The apertured layer specimen is carefully removed such that its longitudinal and lateral extension is maintained to avoid distortion of the apertures. A cryogenic spray (such as Cyto-Freeze, Control Company, Houston TX, or equivalent) can be used to remove the specimen from the underlying layers if necessary. Five replicate specimens obtained from five substantially similar articles are prepared for analysis. An apertured substrate raw material is prepared for testing by extending or activating it under the same process conditions, and to the same extent, as it would be for use on the absorbent article. The samples are conditioned at about 23 °C ± 2 C° and about 50% ± 2% relative humidity for 2 hours prior to testing.

### Image acquisition:

The ruler is placed on the scanner bed such that it is oriented parallel to the sides of the scanner glass. An image of the ruler (the calibration image) is acquired in reflectance mode at a resolution of 6400 dpi (approximately 252 pixels per mm) and in 8-bit grayscale. The calibration image is saved as an uncompressed TIFF format file. After obtaining the calibration image, the ruler is removed from the scanner glass and all specimens are scanned under the same scanning conditions. An apertured specimen is placed onto the center of the scanner bed, lying flat, with the outward facing surface of the specimen facing the scanner's glass surface. The corners and edges of the specimen are secured such that its original longitudinal and lateral extension, as on the article prior to removal, is restored. The specimen is oriented such that the machine direction (MD) and cross direction (CD) of the apertured specimen layer are aligned parallel with and perpendicular to the sides of the scanner's glass surface, respectively, and that the resulting specimen image has the MD vertically running from top to bottom. The black glass tile is placed on top of the specimen, the scanner lid is closed, and a scanned image of the entire specimen is acquired. The specimen image is saved as an uncompressed TIFF format file. The remaining four replicate specimens are scanned and saved in like fashion. Prior to analysis, all specimen images are cropped to the largest rectangular field of view contained within the apertured region which had been located above the absorbent core of the article.

### Percent Open Area Calculations:

The calibration image file is opened in the image analysis program and a linear distance calibration is performed using the imaged ruler. This distance calibration scale is applied to all subsequent specimen images prior to analysis. A specimen image is in the image analysis program and the distance scale is set using the distance calibration. The 8-bit grayscale image is then converted to a binary image (with "zero" or "black" corresponding to the aperture regions) in the following way: If the histogram of gray level (GL) values (ranging from 0 to 255, one bin with propensity Pᵢ per gray level i) has exactly two local maxima, the threshold gray level value t is defined as that value for which Pₜ₋₁ > Pt and Pₜ ≤ Pₜ₊₁. If the histogram has greater than two local maxima, the histogram is iteratively smoothed using a windowed arithmetic mean of size 3, and this smoothing is performed iteratively until exactly two local maxima exist. The threshold gray level value t is defined as that value for which Pₜ₋₁ > Pt and Pₜ ≤ Pₜ₊₁. This procedure identifies the gray level (GL) value for the minimum population located between the dark pixel peak of the aperture holes and the lighter pixel peak of the specimen material. If the histogram contains either zero or one local maximum, the method cannot proceed further, and no output parameters are defined.

Each of the discrete aperture regions is analyzed using the image analysis program. All individual aperture areas are measured and recorded to the nearest 0.01 mm², including partial apertures along the edges of the image. The aperture areas that include whole and partial apertures are summed in area. This sum is then divided by the total area included in the image. This value is multiplied by 100% and reported as the open area to the nearest 0.01%.

The remaining four specimen images are analyzed similarly. The arithmetic mean percent open area values for the five replicate specimens is calculated and reported to the nearest 0.01%.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (10) comprising:
a liquid permeable topsheet (26);
a liquid impermeable backsheet (28);
an absorbent core (30) positioned at least partially between the topsheet (26) and the backsheet (28); and
an outer cover material (40) or a landing zone (44) comprising an embossed pattern;
wherein a garment-facing surface of the backsheet (1300) comprises one or more graphics (1302);
wherein a portion of the embossed pattern overlaps a portion of the one or more graphics;
wherein the portion of the one or more graphics (1302) exhibits a first L*, a*, b* color value when measured through a densified region (1602) of the embossed pattern of the outer cover material (40) or landing zone (44), according to the Backsheet Laminate Color Test Method; and
wherein the portion of the one or more graphics exhibits a second, different L*, a*, b* color value when measured through a non-densified region (1604) of the outer cover material (40) or landing zone (44), according to the Backsheet Laminate Color Test Method;
wherein a wearer-facing surface of the backsheet (1300) comprises a wetness indicator (80), and wherein a zone of different color or different graphics or a zone of no color or no graphics at least partially surrounds the wetness indicator (80).

2. The absorbent article of Claim 1, wherein the embossed pattern forms at least 1 and less than 40 recognizable, discrete indicia.

3. The absorbent article of Claim 2, wherein an entire area of at least one recognizable, discrete indicium is overlapped by the portion of the one or more graphics (1302).

4. The absorbent article of any one of the preceding claims, wherein the outer cover material (40) or the landing zone (44) comprises a densified region (1602) area in the range of about 5% to about 30%, relative to an entire area of the outer cover material (40) or the landing zone (44).

5. The absorbent article of any one of the preceding claims, wherein the first L*, a*, b* color value has an L* value in the range of about 5 to about 95, an a* value in the range of about -90 to about 90, and a b* value in the range of about -90 to about 90, when measured through the densified region (1602) of the embossed pattern of the outer cover material (40) or the landing zone (44), according to the Backsheet Laminate Color Test Method.

6. The absorbent article of any one of the preceding claims, wherein the second L*, a*, b* color value has a L* value in the range of about 5 to about 95, an a* value in the range of about - 90 to about 90, and a b* value in the range of about -90 to about 90, when measured through the non-densified region (1604) of the outer cover material (40) or the landing zone (44), according to the Backsheet Laminate Color Test Method.

7. The absorbent article of Claim 2, wherein a single recognizable, discrete indicium has an area in the range of about 52 mm² to about 7,500 mm².

8. The absorbent article of any one of Claims 2 or 7, wherein the recognizable, discrete indicia comprises an animal, a logo, a brand name, a word, a symbol, a character, or an icon.

9. The absorbent article of any one of Claims 2, 7, or 8, wherein a single graphic of the one or more graphics (1302) has an individual graphic area, wherein a single recognizable, discrete indicium has an individual indicium area, and wherein the individual indicium area is smaller than the individual graphic area.

10. The absorbent article of any one of the preceding claims, wherein a delta E between the first L*, a*, b* color value and the second L*, a*, b* color value is at least 2, preferably between about 2 and about 19, according to the Backsheet Laminate Color Test Method.

11. The absorbent article of any one of the preceding claims, wherein the portion of the one or more graphics (1302) exhibits a chroma value in the range of about 8 to about 130, according to the Backsheet Graphic Color Test Method.

12. The absorbent article of any one of the preceding claims, wherein the portion of the one or more graphics (1302) exhibits an L*value of less than 95, according to the Backsheet Graphic Color Test Method.

13. The absorbent article of any one of the preceding claims, wherein the garment-facing surface of the backsheet (116) comprises a stitch-like pattern (2100) that is free from overlap with the one or more graphics.

14. The absorbent article of any one of the preceding claims, wherein the garment-facing surface of the backsheet (1300) has a graphic-free area of about 5% to about 85%, relative to an entire area of the garment-facing surface of the backsheet (1300).

## Patentansprüche

1. Absorptionsartikel (10), umfassend:
eine flüssigkeitsdurchlässige Oberschicht (26);
eine flüssigkeitsundurchlässige Unterschicht (28);
einen Absorptionskern (30), der wenigstens teilweise zwischen der Oberschicht (26) und der Unterschicht (28) angeordnet ist; und
ein Außenmantelmaterial (40) oder eine Auftreffzone (44), umfassend ein Prägemuster;
wobei eine bekleidungsseitige Oberfläche der Unterschicht (1300) eine oder mehrere Grafiken (1302) umfasst;
wobei ein Abschnitt des Prägemusters einen Abschnitt der einen oder der mehreren Grafiken überlappt;
wobei der Abschnitt der einen oder der mehreren Grafiken (1302) einen ersten L*-, a*-, b*-Farbwert vorweist, wenn durch eine verdichtete Region (1602) des Prägemusters des Außenmantelmaterials (40) oder der Auftreffzone (44) nach dem Unterschichtlaminatfarbprüfverfahren gemessen; und
wobei der Abschnitt der einen oder der mehreren Grafiken einen zweiten, unterschiedlichen L*-, a*-, b*-Farbwert vorweist, wenn durch eine nicht verdichtete Region (1604) des Außenmantelmaterials (40) oder der Auftreffzone (44) nach dem Unterschichtlaminatfarbprüfverfahren gemessen;
wobei eine trägerseitige Oberfläche der Unterschicht (1300) einen Nässeindikator (80) umfasst und wobei ein Bereich einer unterschiedlichen Farbe oder unterschiedlicher Grafiken oder ein Bereich ohne Farbe oder ohne Grafiken den Nässeindikator (80) wenigstens teilweise umgibt.

2. Absorptionsartikel nach Anspruch 1, wobei das Prägemuster wenigstens 1 und weniger als 40 erkennbare, diskrete Markierungen ausbildet.

3. Absorptionsartikel nach Anspruch 2, wobei eine Gesamtfläche wenigstens einer erkennbaren, diskreten Markierung durch den Abschnitt der einen oder der mehreren Grafiken (1302) überlappt ist.

4. Absorptionsartikel nach einem der vorstehenden Ansprüche, wobei das Außenmantelmaterial (40) oder die Auftreffzone (44) eine verdichtete Region (1602) Fläche in dem Umfang von etwa 5 % bis etwa 30 %, relativ zu einer Gesamtfläche des Außenmantelmaterials (40) oder der Auftreffzone (44), umfasst.

5. Absorptionsartikel nach einem der vorstehenden Ansprüche, wobei der erste L*-, a*-, b*-Farbwert einen L*-Wert in dem Umfang von etwa 5 bis etwa 95, einen a*-Wert in dem Umfang von etwa -90 bis etwa 90 und einen b*-Wert in dem Umfang von etwa -90 bis etwa 90 aufweist, wenn durch die verdichtete Region (1602) des Prägemusters des Außenmantelmaterials (40) oder der Auftreffzone (44) nach dem Unterschichtlaminatfarbprüfverfahren gemessen.

6. Absorptionsartikel nach einem der vorstehenden Ansprüche, wobei der zweite L*-, a*-, b*-Farbwert einen L*-Wert in dem Umfang von etwa 5 bis etwa 95, einen a*-Wert in dem Umfang von etwa -90 bis etwa 90 und einen b*-Wert in dem Umfang von etwa -90 bis etwa 90 aufweist, wenn durch die nicht verdichtete Region (1604) des Außenmantelmaterials (40) oder der Auftreffzone (44) nach dem Unterschichtlaminatfarbprüfverfahren gemessen.

7. Absorptionsartikel nach Anspruch 2, wobei eine einzelne erkennbare, diskrete Markierung eine Fläche in dem Umfang von etwa 52 mm² bis etwa 7.500 mm² aufweist.

8. Absorptionsartikel nach einem der Ansprüche 2 oder 7, wobei die erkennbare, diskrete Markierung ein Tier, ein Logo, einen Markennamen, ein Wort, ein Symbol, ein Schriftzeichen oder ein Icon umfasst.

9. Absorptionsartikel nach einem der Ansprüche 2, 7 oder 8, wobei eine einzelne Grafik der einen oder der mehreren Grafiken (1302) eine individuelle Grafikfläche aufweist, wobei eine einzelne erkennbare, diskrete Markierung eine individuelle Markierungsfläche aufweist und wobei die individuelle Markierungsfläche kleiner als die individuelle Grafikfläche ist.

10. Absorptionsartikel nach einem der vorstehenden Ansprüche, wobei ein Delta E zwischen dem ersten L*-, a*-, b*-Farbwert und dem zweiten L*-, a*-, b*-Farbwert nach dem Unterschichtlaminatfarbprüfverfahren wenigstens 2, vorzugsweise zwischen etwa 2 und etwa 19, beträgt.

11. Absorptionsartikel nach einem der vorstehenden Ansprüche, wobei der Abschnitt der einen oder der mehreren Grafiken (1302) nach dem Unterschichtgrafikfarbprüfverfahren einen Farbsättigungswert in dem Umfang von etwa 8 bis etwa 130 vorweist.

12. Absorptionsartikel nach einem der vorstehenden Ansprüche, wobei der Abschnitt der einen oder der mehreren Grafiken (1302) nach dem Unterschichtgrafikfarbprüfverfahren einen L*-Wert von weniger als 95 vorweist.

13. Absorptionsartikel nach einem der vorstehenden Ansprüche, wobei die bekleidungsseitige Oberfläche der Unterschicht (116) ein stichartiges Muster (2100) umfasst, das frei von Überlappung mit der einen oder den mehreren Grafiken ist.

14. Absorptionsartikel nach einem der vorstehenden Ansprüche, wobei die bekleidungsseitige Oberfläche der Unterschicht (1300) eine grafikfreie Fläche von etwa 5 % bis etwa 85 %, relativ zu einer Gesamtfläche der bekleidungsseitigen Oberfläche der Unterschicht (1300), aufweist.

## Revendications

1. Article absorbant (10) comprenant :
une feuille de dessus perméable aux liquides (26) ;
une feuille de fond imperméable aux liquides (28) ;
une âme absorbante (30) positionnée au moins partiellement entre la feuille de dessus (26) et la feuille de fond (28) ; et
un matériau de couverture externe (40) ou une zone de réception (44) comprenant un motif gaufré ;
dans lequel une surface faisant face au vêtement de la feuille de fond (1300) comprend un ou plusieurs graphismes (1302) ;
dans lequel une partie du motif gaufré chevauche une partie du ou des graphismes ;
dans lequel la partie du ou des graphismes (1302) présente une première valeur de couleur L*, a*, b* lorsqu'elle est mesurée à travers une région densifiée (1602) du motif gaufré du matériau de couverture externe (40) ou de la zone de réception (44), selon la méthode d'essai de couleur de stratifié de feuille de fond ; et
dans lequel la partie du ou des graphismes présente une seconde valeur de couleur L*, a*, b* différente lorsqu'elle est mesurée à travers une région non densifiée (1604) du matériau de couverture externe (40) ou de la zone de réception (44), selon la méthode d'essai de couleur de stratifié de feuille de fond ;
dans lequel une surface faisant face au porteur de la feuille de fond (1300) comprend un indicateur d'humidité (80), et dans lequel une zone de couleur différente ou de graphismes différents ou une zone d'absence de couleur ou d'absence de graphismes entoure au moins partiellement l'indicateur d'humidité (80).

2. Article absorbant selon la revendication 1, dans lequel le motif gaufré forme au moins 1 et moins de 40 indications distinctes reconnaissables.

3. Article absorbant selon la revendication 2, dans lequel une zone entière d'au moins une indication distincte reconnaissable est chevauchée par la partie du ou des graphismes (1302).

4. Article absorbant selon l'une quelconque des revendications précédentes, dans lequel le matériau de couverture externe (40) ou la zone de réception (44) comprend une zone de région densifiée (1602) dans la plage d'environ 5 % à environ 30 %, par rapport à une zone entière du matériau de couverture externe (40) ou de la zone de réception (44).

5. Article absorbant selon l'une quelconque des revendications précédentes, dans lequel la première valeur de couleur L*, a*, b* a une valeur L* comprise dans la plage d'environ 5 à environ 95, une valeur a* comprise dans la plage d'environ - 90 à environ 90, et une valeur b* comprise dans la plage d'environ -90 à environ 90, lorsqu'elles sont mesurées à travers la région densifiée (1602) du motif gaufré du matériau de couverture externe (40) ou de la zone de réception (44), selon la méthode d'essai de couleur de stratifié de feuille de fond.

6. Article absorbant selon l'une quelconque des revendications précédentes, dans lequel la seconde valeur de couleur L*, a*, b* a une valeur L* comprise dans la plage d'environ 5 à environ 95, une valeur a* comprise dans la plage d'environ - 90 à environ 90, et une valeur b* comprise dans la plage d'environ -90 à environ 90, lorsqu'elles sont mesurées à travers la région non densifiée (1604) du matériau de couverture externe (40) ou de la zone de réception (44), selon la méthode d'essai de couleur de stratifié de feuille de fond.

7. Article absorbant selon la revendication 2, dans lequel une indication distincte reconnaissable unique a une aire comprise dans la plage d'environ 52 mm² à environ 7 500 mm².

8. Article absorbant selon l'une quelconque des revendications 2 ou 7, dans lequel l'indication distincte reconnaissable comprend un animal, un logo, un nom de marque, un mot, un symbole, un personnage, ou une icône.

9. Article absorbant selon l'une quelconque des revendications 2, 7 ou 8, dans lequel un graphisme unique du ou des graphismes (1302) a une zone de graphisme individuelle, dans lequel une indication distincte reconnaissable unique a une zone d'indication individuelle, et dans lequel la zone d'indication individuelle est plus petite que la zone de graphisme individuelle.

10. Article absorbant selon l'une quelconque des revendications précédentes, dans lequel un delta E entre la première valeur de couleur L*, a*, b* et la seconde valeur de couleur L*, a*, b* est au moins de 2, de préférence est comprise entre environ 2 et environ 19, selon la méthode d'essai de couleur de stratifié de feuille de fond.

11. Article absorbant selon l'une quelconque des revendications précédentes, dans lequel la partie du ou des graphismes (1302) présente une valeur de chrominance comprise dans la plage d'environ 8 à environ 130, selon la méthode d'essai de couleur de graphisme de feuille de fond.

12. Article absorbant selon l'une quelconque des revendications précédentes, dans lequel la partie du ou des graphismes (1302) présente une valeur L* inférieure à 95, selon la méthode d'essai de couleur de graphisme de feuille de fond.

13. Article absorbant selon l'une quelconque des revendications précédentes, dans lequel la surface faisant face au vêtement de la feuille de fond (116) comprend un motif de type points de couture (2100) qui est exempt de chevauchement avec le ou les graphismes.

14. Article absorbant selon l'une quelconque des revendications précédentes, dans lequel la surface faisant face au vêtement de la feuille de fond (1300) a une zone sans graphisme d'environ 5 % à environ 85 %, par rapport à une zone entière de la surface faisant face au vêtement de la feuille de fond (1300).
